(19) Europäisches Patentamt
European Patent Office
Office européen des brevets

(11) **EP 3 258 284 B1**

(12) **EUROPÄISCHE PATENTSCHRIFT**

(45) Veröffentlichungstag und Bekanntmachung des Hinweises auf die Patenterteilung:
**21.07.2021 Patentblatt 2021/29**

(51) Int Cl.:
***G01R 33/28*** *(2006.01)*     ***A61B 5/055*** *(2006.01)*
***G01R 33/54*** *(2006.01)*

(21) Anmeldenummer: **17158047.5**

(22) Anmeldetag: **27.02.2017**

(54) **BETRIEB EINER MAGNETRESONANZVORRICHTUNG UNTER BERÜCKSICHTIGUNG VON IMPLANTAT-TRÄGERN**

OPERATING A MAGNETIC RESONANCE DEVICE WITH REGARD TO PEOPLE WEARING IMPLANTS

FONCTIONNEMENT D'UN APPAREIL À RÉSONANCE MAGNÉTIQUE EN TENANT COMPTE DES PERSONNES PORTANT DES IMPLANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.04.2016 DE 102016206398**

(43) Veröffentlichungstag der Anmeldung:
**20.12.2017 Patentblatt 2017/51**

(73) Patentinhaber: **Siemens Healthcare GmbH**
**91052 Erlangen (DE)**

(72) Erfinder:
- **Bielmeier, Wolfgang**
  **91056 Erlangen (DE)**
- **Brinker, Gerhard**
  **91054 Erlangen (DE)**
- **Campagna, Swen**
  **91238 Engelthal (DE)**
- **Demharter, Nikolaus**
  **91077 Dormitz (DE)**
- **Erbe, Bernd**
  **91056 Erlangen (DE)**
- **Gebhardt, Matthias**
  **91052 Erlangen (DE)**
- **Nistler, Jürgen**
  **91056 Erlangen (DE)**
- **Paul, Dominik**
  **91088 Bubenreuth (DE)**
- **Prinz, Carsten**
  **91083 Baiersdorf (DE)**
- **Ruyters, Gudrun**
  **91058 Erlangen (DE)**
- **Stöcker, Stephan**
  **91083 Baiersdorf (DE)**
- **Vester, Markus**
  **90471 Nürnberg (DE)**

(56) Entgegenhaltungen:
DE-A1-102006 016 043     DE-U1-202008 018 452
US-A1- 2007 265 685     US-A1- 2012 086 449

EP 3 258 284 B1

# EP 3 258 284 B1

## Beschreibung

Die Erfindung betrifft ein Verfahren zum Betrieb einer Magnetresonanzvorrichtung unter Berücksichtigung von Implantat-Trägern mittels einer Sicherheitseinheit und eine Magnetresonanzvorrichtung.

Bei einer Magnetresonanzuntersuchung, d.h. bei einer Durchführung einer Magnetresonanztomographie (MRT, engl. Magnetic Resonance Imaging, MRI), werden zur Erfassung von Magnetresonanzsignalen durch eine Magnetresonanzvorrichtung üblicherweise elektromagnetische Felder, insbesondere Gradientenfelder und Hochfrequenzfelder gemäß einem Messprotokoll eingesetzt. Zur Erzeugung der Gradientenfelder weist die Magnetresonanzvorrichtung üblicherweise eine Gradientenspuleneinheit mit zumindest einer Gradientenspule auf. Ferner umfasst die Magnetresonanzvorrichtung meist eine Hochfrequenzantenneneinheit, mit der Hochfrequenzfelder zu einer Anregung von Atomkernen erzeugt werden können.

Die Gradientenfelder können periphere Nervenstimulationen (PNS) und die Hochfrequenzfelder Erwärmungen bewirken. Daher fordern Normungsgremien, wie z.B. IEC 60601-2-33, dass diese PNS und/oder Erwärmungen überwacht und begrenzt werden. In der Vergangenheit waren Patienten mit Implantaten in der Regel von einer Magnetresonanzuntersuchung ausgeschlossen. Die Entwicklung neuer, bedingt MR-kompatibler Implantate, die gemäß der Norm IEC 60601-2-33 mit "Fixed Parameter Option: Basic" (FPO:B) gekennzeichnet werden können, ermöglicht Magnetresonanzuntersuchung bei Implantat-Trägern zuzulassen, wenn bestimmte Bedingungen erfüllt werden.

Typische bedingt MR-kompatible Implantate können beispielsweise Herzschrittmacher, Defibrillatoren und weitere Implantate, wie etwa zur Verabreichung von Medikamenten oder zur tiefen Hirnstimulation oder zur Stimulation der Wirbelsäule. Bei einer Untersuchung eines Trägers eines solchen Implantats unter Anwendung der Norm IEC 60601-2-33 ist sicherzustellen, dass die Magnetresonanzvorrichtung in einem sicheren Betriebsmodus betrieben wird, um die Gesundheit des Implantat-Trägers nicht zu gefährden. Üblicherweise bedingt der sichere Betriebsmodus eine Einschränkung der Leistungsfähigkeit der Magnetresonanzvorrichtung, so dass der sichere Betriebsmodus auch als ein eingeschränkter Betriebsmodus bezeichnet werden kann. So soll gemäß der Norm IEC 60601-2-33 insbesondere ein effektiver $B_1^+$-Wert $B_{1\,rms}^+$, ein maximaler $B_i^+$-Wert $B_{1\,peak}^+$, ein Betrag einer effektiven Änderungsrate eines B-Werts $(|dB/dt|_{rms})_{FPO}$ und ein Betrag einer maximalen Änderungsrate $(|dB/dt|_{peak})_{FPO}$ nicht überschritten werden.

Die Druckschrift US 2007/265685 A1 offenbart ein Verfahren zur Erkennung von Implantaten bei der Durchführung von Magnetresonanzuntersuchungen.

Die Druckschrift DE 10 2006 016043 A1 offenbart ein Verfahren zur Feststellung einer möglichen Gefährdung durch ein Gerät.

Die Druckschrift DE 20 2008 018452 U1 offenbart ein Magnetresonanz-Sicherheitsüberwachungssystem, das einen potentiell unsicheren Zustand erkennt und eine Korrektur der Magnetresonanzanregung durchführt.

Die Druckschrift US 2012/086449 A1 offenbart ein Verfahren für umfassende Sicherheitsprüfungen implantierbarer Einrichtungen und Patientensicherheitsüberwachung.

Der Erfindung liegt die Aufgabe zugrunde, einen sicheren Betrieb einer Magnetresonanzvorrichtung unter Berücksichtigung von Implantat-Trägern zu ermöglichen. Insbesondere sollen dabei die Anforderungen der Norm IEC 60601-2-33 erfüllt werden. Die Aufgabe wird durch die Merkmale der unabhängigen Ansprüche gelöst. Vorteilhafte Ausgestaltungen sind in den Unteransprüchen beschrieben.

Demnach wird ein Verfahren zum Betrieb einer Magnetresonanzvorrichtung unter Berücksichtigung von Implantat-Trägern mittels einer Sicherheitseinheit vorgeschlagen. Dabei umfasst die Magnetresonanzvorrichtung einen ersten Teil und einen zweiten Teil, wobei der erste Teil getrennt von dem zweiten Teil betrieben wird und die Sicherheitseinheit umfasst. Der zweite Teil weist eine Systemsteuereinheit zur Steuerung der Magnetresonanzvorrichtung auf und die Sicherheitseinheit ist ausgebildet, Kontrollsignale an die Systemsteuereinheit des zweiten Teils zu senden. Bei einer Untersuchung eines Implantat-Trägers kontrolliert die Sicherheitseinheit, dass die Magnetresonanzvorrichtung in einem eingeschränkten Betriebsmodus Implantats-konforme Grenzwerte einhält. Dabei wird der erste Teil mit Software, die Sicherheitsanforderungen gemäß einer ersten Sicherheitskategorie erfüllt und der zweite Teil mit Software, die Sicherheitsanforderungen gemäß einer zweiten Sicherheitskategorie erfüllt, betrieben, wobei die erste Sicherheitskategorie höhere Sicherheitsanforderungen als die zweite Sicherheitskategorie aufweist.

Die Implantats-konforme Grenzwerte können beispielsweise durch die Norm IEC 60601-2-33, z.B. Ausgabe 3.2. 2015-06, vorgegeben werden. Diese sieht insbesondere vor, dass folgende Grenzwerte eingehalten werden: $B_{i\,peak}^+ \leq 30\ \mu T$, $B_{1\,rms}^+ \leq 3,2\ \mu T$, $(|dB/dt|_{peak})_{FPO} \leq 100$ T/s $(|dB/dt|_{rms})_{FPO} \leq 56$ T/s. Der Implantat-Träger ist vorzugsweise ein Patient, der mindestens ein bedingt MR-kompatibles Implantat, das z.B. mit "FPO:B" gekennzeichnet ist, aufweist.

Der getrennte Betrieb von ersten und zweiten Teil kann insbesondere bedeuten, dass der zweite Teil keinen Einfluss auf eine oder mehrere, vorzugsweise alle, sicherheitsrelevante Funktionen des ersten Teils besitzt. Der getrennte Betrieb von ersten und zweiten Teil schließt also nicht aus, dass der erste und zweite Teil miteinander wechselwirken. Zudem kann der getrennte Betrieb von ersten und zweiten Teil insbesondere bedeuten, dass der zweite Teil zwar Einfluss auf eine oder mehrere sicherheitsrelevante Funktionen des ersten Teils besitzt, aber der erste Teil einen Mechanismus aufweist, die im Falle einer sicherheitsrelevanten Beeinflussung des ersten Teils durch den zweiten Teil eine Gefährdung und/oder Verletzung des zu untersuchenden Implantat-Trägers verhindert. Vorzugsweise weist der erste und der zweite Teil jeweils mindestens einen eigeständigen Prozessor und/oder ein eigenständiges Betriebssystem auf, das beispielsweise auf dem mindestens einen eigenständigen Prozessor ausgeführt werden kann. Vorzugsweise weist die Sicherheitseinheit einen oder mehrere Prozessoren auf, die unabhängig von etwaigen Prozessoren des zweiten Teils betrieben werden.

Erfindungsgemäß erfüllt der erste Teil höhere Sicherheitsanforderungen als der zweite Teil. Da die Untersuchung eines Implantat-Trägers durch die Sicherheitseinheit, die vom ersten Teil umfasst wird, kontrolliert wird, ist der erste Teil nach besonders hohen Sicherheitskriterien ausgebildet, um das Risiko einer untersuchungsbedingten Verletzung des Implantat-Trägers so klein wie möglich zu halten.

Erfindungsgemäß wird der erste Teil mit Software gemäß einer ersten Sicherheitskategorie und der zweite Teil mit Software gemäß einer zweiten Sicherheitskategorie betrieben, wobei die erste Sicherheitskategorie höhere Sicherheitsanforderungen als die zweite Sicherheitskategorie aufweist.

Bevorzugt erfüllt die erste Sicherheitskategorie die Sicherheitsklasse C und die zweite Sicherheitskategorie die Sicherheitsklassen A und/oder B der Norm IEC 62304. Die Sicherheitsklasse C sieht üblicherweise bestimmte Designanforderungen vor, wie beispielsweise einen Nachweis, dass eine vorgegebene Architektur korrekt implementiert ist, eine Beschreibung der Funktionalität, der Schnittstellen zwischen Hardware und Software und/oder des Datenflusses zu anderen Komponenten.

Ferner sieht die Sicherheitsklasse C üblicherweise bestimmte Implementierungsanforderungen vor, wie beispielsweise dass die Trennung zwischen Komponenten definiert und ihre Effektivität nachgewiesen wird. Ferner werden vorteilhafterweise eine oder mehrere der folgenden Aspekte berücksichtigt, dokumentiert und getestet: Datenfluss und Kontrollfluss, geplante Zuweisung von Ressourcen, Fehlerbehandlung, Initialisierung zu klärender Variablen, zu berücksichtigende Selbstdiagnose, Speichermanagement und Speicherüberlauf, Randbedingungen für die Ausführung.

Die Verwendung einer separat betriebenen Sicherheitseinheit erleichtert die Erfüllung der Anforderungen an die Software gemäß der Sicherheitsklasse C, beispielsweise hinsichtlich des Ressourcen-Sharings, etwa eines Hauptprozessors (engl. central processing unit, CPU) und/oder eines Direktzugriffspeichers (engl. random-access memory, RAM). Die Trennung des ersten und zweiten Teils erlaubt eine einfachere Herstellung des Gesamtsystems, da vorzugsweise nur der erste Teil die erhöhten Sicherheitsanforderungen gemäß Sicherheitsklasse C erfüllt, wohingegen es für den zweiten Teil auch ausreichen kann, eine geringere Sicherheitsklasse zu erfüllen.

Vorteilhafterweise erfolgen die Erzeugung und/oder Übertragung von Signalen innerhalb des ersten Teils, idealerweise ausschließlich, mittels Hardware. Dadurch können Anforderungen der Sicherheitsklasse C erfüllt werden.

Ferner ist denkbar, dass die Sicherheitsanforderungen an die Programmmittel des ersten Teils ganz oder teilweise von Sicherheitsklasse C auf die Sicherheitsklasse B reduziert werden, indem ein sicherheitssicherndes Konzept als Hardware implementiert wird und die Programmmittel lediglich die Hardwarefunktionen überwachen und/oder unterstützen.

Eine Ausführungsform des Verfahrens sieht vor, dass der erste Teil eine Schalteinheit umfasst. Vorzugsweise wird der Sicherheitseinheit durch die Schalteinheit eine Schaltinformation aus mehreren möglichen Schaltinformationen bereitgestellt. Dabei wird abhängig von der bereitgestellten Schaltinformation durch die Sicherheitseinheit ein Betriebsmodus aus mehreren möglichen Betriebsmodi eingestellt.

Die Einstellung des Betriebsmodus ist dabei vorzugsweise explizit sicherheitsorientiert, d.h. eine unbeabsichtigte Aktivierung des eingeschränkten Betriebsmodus führt zu keinen Sicherheitsrisiken für den Patienten, sondern vermindert allenfalls die Verfügbarkeit der Magnetresonanzvorrichtung, da möglicherweise nicht mehr alle sonst ausführbaren Messprotokolle verwendet werden können. Andererseits sollte eine unbeabsichtigte Deaktivierung des eingeschränkten Betriebsmodus vermieden werden, wenn ein eingeschränkter Betriebsmodus aus Sicherheitsgründen erforderlich ist.

Die Schalteinheit kann eine Einheit sein, mit der ein gewünschter Betriebsmodus der Magnetresonanzvorrichtung angegeben werden kann, insbesondere ob eine Untersuchung im eingeschränkten Betriebsmodus durchgeführt werden soll.

Die Bereitstellung der Schaltinformationen und/oder des Patienteninformation erfolgt vorzugsweise in mit Hilfe eines, insbesondere elektronischen und/oder elektrischen, Signals. Insbesondere die Bereitstellung der Schaltinformationen erfolgt mit Hilfe einer Hardware-Verbindung, um eine hohe Sicherheit zu gewährleisten.

Insbesondere kann die Schalteinheit einen Schalter und/oder einen Taster umfassen.

Ein Schalter weist üblicherweise zu einem Zeitpunkt entweder einen offenen oder einen geschlossenen Schaltzustand auf. Beispielsweise ist in einem geschlossenen Schaltzustand ein Stromkreis geschlossen und in einem offenen Schaltzustand unterbrochen. Eine Schaltinformation eines Schalters kann beispielsweise sein Schaltzustand zu einem bestimmten Zeitpunkt und/oder ein Wechsel eines Schaltzustands sein, insbesondere von einem offenen zu einem geschlossenen Zustand oder von einem geschlossenen Zustand zu einem offenen Zustand.

Die Schaltinformation eines Tasters kann beispielsweise ein Schaltimpuls sein, der durch eine Betätigung des Tasters ausgelöst wird. Beispielsweise kann durch Betätigung eines Tasters ein Stromkreis geschlossen oder unterbrochen werden.

Die Bereitstellung der Schaltinformation kann durch ein Bedienpersonal der Magnetresonanzvorrichtung durch Betätigung der Schalteinheit ausgelöst werden, beispielsweise indem er einen Schalter umlegt und/oder einen Taster drückt.

Eine Ausführungsform des Verfahrens sieht vor, dass der zweite Teil eine Patientenregistrierungseinheit umfasst, wobei der Sicherheitseinheit durch die Patientenregistrierungseinheit eine Patientenregistrierungsinformation bereitgestellt wird, wobei ein Wechsel des Betriebsmodus zumindest teilweise durch die bereitgestellte Patientenregistrierungsinformation ausgelöst wird.

Die Patientenregistrierungseinheit gibt vorzugsweise mit Hilfe der Patientenregistrierungsinformation eine Information über einen Patientenwechsel an die Sicherheitseinheit weiter. Wenn beispielsweise ein vorheriger Patient die Magnetresonanzvorrichtung verlassen hat und die Untersuchung eines nachfolgenden Patienten ansteht, sendet die Patientenregistrierungseinheit die Patientenregistrierungsinformation an die Sicherheitseinheit. Mit anderen Worten signalisiert die Patientenregistrierungsinformation der Sicherheitseinheit, dass im weiteren Verlauf ein neuer Patient untersucht werden soll.

Vorzugsweise veranlasst die Patientenregistrierungsinformation eine Auswertung der bereitgestellten Schaltinformation, d.h. sie dient als Triggersignal für eine derartige Auswertung. Abhängig von dem Ergebnis dieser Auswertung erfolgt vorzugsweise die Einstellung des Betriebsmodus aus den mehreren möglichen Betriebsmodi. Die Einstellung des Betriebsmodus kann insbesondere umfassen, dass ein momentaner Betriebsmodus beibehalten wird und/oder dass der momentane Betriebsmodus verändert wird, d.h. dass ein anderer der mehreren möglichen Betriebsmodi eingestellt wird.

Durch eine durch die Patientenregistrierungsinformation ausgelöste Berücksichtigung der Schaltinformation wird die Wahrscheinlichkeit erhöht, dass ein geeigneter, insbesondere ausreichend sicherer, Betriebsmodus eingestellt wird. So kann das Risiko reduziert werden, dass eine etwaige Fehlfunktion aus dem zweiten Teil, der möglicherweise geringere Sicherheitsanforderungen erfüllt als der erste Teil, zu einem unsicheren Betrieb der Magnetresonanzvorrichtung führt.

Eine Ausführungsform des Verfahrens sieht vor, dass die mehreren möglichen Betriebsmodi einen uneingeschränkten Betriebsmodus und den eingeschränkten Betriebsmodus umfassen und die mehreren möglichen Schaltinformationen eine aktive Schaltinformation und eine passive Schaltinformation umfassen. Dabei wird der uneingeschränkte Betriebsmodus eingestellt, wenn zum Zeitpunkt der Bereitstellung der Patientenregistrierungsinformation die bereitgestellte Schaltinformation die passive Schaltinformation ist.

Vorzugsweise ist es beispielsweise für einen Wechsel vom eingeschränkten Betriebszustand in den uneingeschränkten Betriebszustand nicht bereits ausreichend, wenn das Bedienpersonal die Schalteinheit betätigt, so dass die passive Schaltinformation bereitgestellt wird, sondern darüber hinaus ist als Triggersignal eine Übertragung des Patientenregistrierungsinformation an die Sicherheitseinheit notwendig.

Üblicherweise ist unter einem uneingeschränkten Betriebsmodus ein Betriebsmodus zu verstehen, bei dem keine Rücksicht auf etwaige Implantate genommen wird, d.h. es werden beispielsweise keine Vorgaben gemäß FPO:B beachtet.

Unter einer aktiven Schaltinformation kann hier eine Information verstanden werden, wonach eine Untersuchung im eingeschränkten Betriebsmodus erwünscht ist. Dagegen kann unter einer passiven Schaltinformation eine Information verstanden werden, wonach eine Untersuchung im uneingeschränkten Betriebsmodus erwünscht ist.

Eine Variante des Verfahrens sieht vor, dass der eingeschränkte Betriebsmodus eingestellt wird, wenn zum Zeitpunkt der Bereitstellung der Patientenregistrierungsinformation die bereitgestellte Schaltinformation die aktive Schaltinformation ist. Ein möglicher Wechsel von dem uneingeschränkten Betriebsmodus in dem eingeschränkten Betriebsmodus findet demnach nur dann statt, wenn von der Sicherheitseinheit auch eine Patientenregistrierungsinformation empfangen wird. Ein Wechsel in den eingeschränkten Betriebsmodus erfolgt gemäß dieser Variante also in analoger Weise wie ein Wechsel in den uneingeschränkten Betriebsmodus, nämlich bei Bereitstellung einer Patientenregistrierungsinformation.

Eine alternative Variante sieht vor, dass der eingeschränkte Betriebsmodus eingestellt wird, wenn die bereitgestellte Schaltinformation die aktive Schaltinformation ist, also insbesondere unabhängig von etwaigen Patientenregistrierungsinformationen. Das bedeutet, dass eine Aktivierung des eingeschränkten Betriebsmodus jederzeit möglich ist, eine Deaktivierung aber nur bei Bereitstellung der Patientenregistrierungsinformationen.

Vorzugsweise umfasst der erste Teil eine Anzeigeeinheit, die den Betriebsmodus anzeigt. Bevorzugt umfasst die Anzeigeeinheit ein visuelles Anzeigemittel, wie beispielsweise eine Lichtdiode (engl. light emitting diode, LED). Wenn z.B. der eingeschränkte Betriebsmodus aktiv ist, leuchtet die LED, anderenfalls nicht. Dies ermöglicht eine komfortable Bedienung und Überwachung der Sicherheitseinheit, da sich das Bedienpersonal schnell und einfach über den aktuellen Betriebszustand informieren kann und gegebenenfalls darauf reagieren kann. Bevorzugt erfolgt eine Ansteuerung der Anzeigeeinheit mit Hilfe einer Hardware-Verbindung.

Eine Ausführungsform des Verfahrens sieht vor, dass der Sicherheitseinheit während der Untersuchung Sicherheitsmessdaten bereitgestellt werden, anhand derer die Kontrolle auf Einhaltung der Implantats-konformen Grenzwerte erfolgt.

Die Sicherheitsmessdaten können beispielsweise Daten über Ströme und/oder Spannungen der Hochfrequenzantenneneinheit umfassen, beispielsweise Spannungen an Hochfrequenzspulenanschlüssen. Insbesondere können sie Daten über breitbandige und/oder schmalbandige hochfrequente Anregungssignale umfassen, die von einer Hochfrequenzantenneneinheit der Magnetresonanzvorrichtung während der Untersuchung ausgesendet werden.

Außerdem können die Sicherheitsmessdaten Ströme und/oder Spannungen einer Gradientenspuleneinheit umfassen, insbesondere Gradientenströme, die insbesondere zur Erzeugung von Gradientenfeldern in einer oder mehreren Gradientenspulen angelegt werden.

Vorteilhafterweise sind die Sicherheitsmessdaten geeignet, daraus sicherheitsrelevante Größen wie z.B. $B_1{}^+_{peak}$, $B_1{}^+_{rms}$, $(|dB/dt|_{peak})_{FPO}$ und/oder $(|dB/dt|_{rms})$ abzuleiten, die durch die Magnetresonanzvorrichtung hervorgerufen werden und deren Einhaltung durch die Sicherheiteinheit kontrolliert wird. Es ist aber auch denkbar, dass die Magnetresonanzvorrichtung dezidierte Messvorrichtungen, wie z.B. Pick-Spulen umfasst, durch welche die sicherheitsrelevanten Größen, insbesondere Felder, als Sicherheitsmessdaten zumindest teilweise direkt erfasst werden können.

Bevorzugt werden die bereitgestellten Sicherheitsmessdaten zumindest teilweise verifiziert, um sicherzustellen, dass die Sicherheitsmessdaten korrekt detektiert und übertragen wurden. Zur Verifikation der Messdaten sind beispielsweise eine oder mehrere redundante Messeinheiten vorgesehen, mit der insbesondere redundante Sicherheitsmessdaten erfasst werden können, mit denen ein Teil oder alle bereitgestellten Sicherheitsmessdaten verglichen werden. Ferner ist es denkbar, dass eine oder mehrere Messeinheiten zur zyklischen Kontrolle der Hauptmesseinheiten vorgesehen sind.

Somit kann verhindert werden, dass vorgesehene Sicherheitsalgorithmen fehlschlagen. Beispielsweise kann ein Breitbandvergleich und/oder Schmalbandvergleich, insbesondere zur Laufzeit, durchgeführt werden.

Ferner ist denkbar, dass eine zyklische Redundanzprüfung (engl. cyclic redundancy check, CRC) durchgeführt wird, um zu überprüfen, ob die Sicherheitsmessdaten korrekt übertragen wurden. Dies ist insbesondere dann vorteilhaft, falls die Übertragung der Sicherheitsmessdaten Softwaremittel beinhaltet.

Eine weitere Ausführungsform sieht vor, dass der Sicherheitseinheit mindestens ein Konfigurationsparameterdatensatz bereitgestellt wird, anhand dessen die Kontrolle auf Einhaltung der Implantats-konformen Grenzwerte erfolgt.

Mit Hilfe des mindestens einen Konfigurationsparameterdatensatzes kann die Sicherheitseinheit mit systemspezifischen Parametern konfiguriert werden, so dass die Sicherheitseinheit aus den Sicherheitsmessdaten korrekte sicherheitsrelevante Größen ermitteln kann.

Der mindestens eine Konfigurationsparameterdatensatz umfasst beispielsweise Parameter wie eine Gradientenspulenempfindlichkeit und/oder einen Umrechnungsfaktor, anhand dessen aus einem Spannungswert ein $B_1{}^+$-Wert und/oder aus einem Stromwert ein Änderungsrate eine Magnetfelds dB/dt berechnet werden kann. Üblicherweise unterscheiden sich diese Parameter je nach Bauart der Hochfrequenzantenneneinheit und/oder der Gradientenspuleneinheit.

Eine mögliche Variante des Verfahrens sieht vor, dass der mindestens eine Konfigurationsparameterdatensatz zumindest teilweise im ersten Teil hinterlegt wird. Dadurch kann eine Übermittlung des zumindest einen Konfigurationsparameterdatensatzes vom zweiten Teil mit möglicherweise weniger hohen Sicherheitsanforderungen in den ersten Teil vermieden werden. Dies ermöglicht eine einfache Architektur des Sicherheitskonzepts, da z.B. von vorneherein keine Anforderungen gemäß der Sicherheitsklasse C auf den zweiten Teil übertragen werden müssen und/oder der zweite Teil von solchen Anforderungen nicht betroffen ist.

Beispielsweise kann der mindestens eine Konfigurationsparameterdatensatz von vorneherein so festgelegt werden, dass er für eine Vielzahl von möglichen Konfigurationen von Magnetresonanzvorrichtung gültig ist, so dass idealerweise keine gerätespezifische Konfiguration der Sicherheitseinheit notwendig ist. Die Vielzahl von möglichen Konfigurationen von Magnetresonanzvorrichtungen, insbesondere bezüglich der Hochfrequenzantenneneinheit und/oder der Gradientenspuleneinheit, kann beispielsweise alle möglichen Konfigurationen von Magnetresonanzvorrichtungen eines Herstellers betreffen, die insbesondere zum Betrieb unter Berücksichtigung von Implantat-Trägern vorgesehen sind. Die Vielzahl der möglichen Konfigurationen kann beispielsweise dadurch bedingt sein, dass Low-Cost-Systeme meist anders ausgebildet sind als High-End-Systeme.

Insbesondere kann dabei dem zumindest einen Konfigurationsparameterdatensatz ein ungünstigster Fall (engl. worst case) zugrunde gelegt sein. Dies insbesondere deshalb vorteilhaft, da etwaige Komponenten der Magnetresonanzvorrichtungen, wie beispielsweise die Hochfrequenzantenneneinheit und/oder die Gradientenspuleneinheit, oftmals nicht mit einer Hardware-Identifikation ausgerüstet sind und daher konventioneller Weise in der Regel über Software im Rahmen einer Installation und/oder Inbetriebnahme der Magnetresonanzvorrichtung konfiguriert werden. Um den anderenfalls notwendigen hohen Aufwand zur Gestaltung der Software gemäß einer hohen Sicherheitsklasse, z.B. Sicherheitsklasse C, zu vermeiden, wird vorgeschlagen, Konfigurationsparameterdatensatz als Worst-Case-Datensatz im Sinne der FPO-Grenzen auszulegen. Dies ist besonders effizient, da somit keine gerätespezifische Installation des ersten Teils und/oder der Sicherheitseinheit notwendig ist.

Alternativ kann der mindestens eine Konfigurationsparameterdatensatz für eine spezifische Konfiguration einer Magnetresonanzvorrichtung in den ersten Teil hinterlegt werden, so dass insbesondere kein ungünstigster Fall über eine Vielzahl an möglichen Konfigurationen von Magnetresonanzvorrichtungen betrachtet werden muss. Gerade wenn die Vielzahl an möglichen Konfigurationen von Magnetresonanzvorrichtungen eine große Varianz aufweisen würde, könnte dies möglicherweise zu einem Verlust an Leistungsfähigkeit führen.

Dieser gerätespezifische mindestens eine Konfigurationsparameterdatensatz kann beispielsweise in einen möglichen nichtflüchtigen Speicher, z.B. ein EEPROM-Speicher, des ersten Teils, insbesondere als Firmware, abgespeichert werden. Das Generieren und/oder Speichern des mindestens einen Konfigurationsparameterdatensatz kann im Rahmen der Herstellung der Magnetresonanzvorrichtung erfolgen.

Ferner ist es denkbar, dass der zumindest eine Konfigurationsparameterdatensatz mehrere Konfigurationsparameterdatensätze umfasst, wobei aus den mehreren Konfigurationsparameterdatensätzen ein Konfigurationsparameterdatensatz abhängig von Parametern der Magnetresonanzvorrichtung, wie z.B. Eigenschaften etwaiger Gradientenspulen und/oder anderer Komponenten der Magnetresonanzvorrichtung, ausgewählt wird. Um eine Interaktion zwischen dem ersten Teil und dem zweiten Teil zu vermeiden, sind sowohl die mehreren Konfigurationsparameterdatensätze als auch die Parameter der Magnetresonanzvorrichtung im ersten Teil hinterlegt.

Dadurch kann vorteilhafterweise ein angepasster, vorzugsweise für die jeweilige Ausführung der Magnetresonanzvorrichtung idealer, Konfigurationsparameterdatensatz bereitgestellt werden, ohne dass eine Interaktion zwischen dem ersten und dem zweiten Teil notwendig ist.

Eine weitere Ausführungsform sieht vor, dass der mindestens eine Konfigurationsparameterdatensatz zumindest teilweise von dem zweiten Teil in den ersten Teil übertragen wird. Dies ermöglicht eine externe Konfiguration der Sicherheitseinheit. Vorteilhafterweise ist der Konfigurationsparameterdatensatz auf den spezifischen Typ der Magnetresonanzvorrichtung abgestimmt. Dies ermöglicht eine optimale Anpassung der Sicherheitsvorrichtung und/oder der Kontrolle durch die Sicherheitsvorrichtung an die Ausgestaltung der Magnetresonanzvorrichtung.

Die Übertragung kann insbesondere zur Laufzeit erfolgen, insbesondere nachdem die Sicherheitseinheit gestartet wurde. Dabei können beispielsweise mehrere Konfigurationsparameterdatensätze mittels Programmmitteln gemäß der Sicherheitsklasse A und/oder B übertragen werden, wobei aus den mehreren Konfigurationsparameterdatensätzen ein Konfigurationsparametersatz gemäß der Sicherheitsklasse C ausgewählt wird, z.B. durch manuelle Installation und/oder Verifikation durch zumindest zwei Bediener und/oder Programmmitteln.

Bevorzugt wird der mindestens eine bereitgestellte Konfigurationsparameterdatensatz anhand einer Prüfsumme verifiziert. Die Prüfsumme kann eine Soll-Prüfsumme umfassen, die anhand einer Soll-Geräte-ID und des mindestens einen Konfigurationsparametersatz einen gebildet werden kann. Vorzugsweise ist dabei die Soll-Geräte-ID ausgebildet, einen Typ einer Magnetresonanzvorrichtung und/oder relevanter Komponenten einer Magnetresonanzvorrichtung eindeutig zu identifizieren. Die Soll-Geräte-ID ist vorzugsweise untrennbar mit einem Typ einer Magnetresonanzvorrichtung und/oder relevanter Komponenten verknüpft und/oder unabhängig ermittelbar.

Unter einem Typ können hier insbesondere Ausführungsformen von Magnetresonanzvorrichtungen und/oder relevanter, insbesondere felderzeugender, Komponenten einer Magnetresonanzvorrichtung subsumiert werden, die hinsichtlich der Erzeugung von Gradientenfeldern und/oder Hochfrequenzfeldern gleiche Eigenschaften aufweisen, insbesondere vergleichbare Felder erzeugen. Magnetresonanzvorrichtung und/oder relevanter Komponenten einer Magnetresonanzvorrichtung eines gleichen Typs müssen daher nicht unbedingt baugleich sein. Beispielsweise ist es denkbar, dass zwei Magnetresonanzvorrichtungen eines gleichen Typs in dem oben beschriebenen Sinne sich dadurch unterscheiden, dass sie unterschiedliche Patientenlagerungsvorrichtungen und/oder Benutzerschnittstellen aufweisen.

Anhand des Konfigurationsparameterdatensatzes und/oder der Soll-Prüfsumme und/oder der Soll-Geräte-ID kann ein abgeschlossener Datensatz (engl. self-contained data set) erzeugt werden. Bevorzugt sind der Konfigurationsparameterdatensatz und/oder der abgeschlossene Datensatz im zweiten Teil der Magnetresonanzvorrichtung gespeichert. Der Konfigurationsparameterdatensatz und/oder der abgeschlossene Datensatz kann vom zweiten Teil in den ersten Teil übertragen werden.

Vorzugsweise findet die Verifikation des bereitgestellten mindestens einen Konfigurationsparameterdatensatzes und/oder des abgeschlossenen Datensatzes im ersten Teil statt. Dabei wird vorzugsweise eine Ist-Geräte-ID verwendet, die ausgebildet ist, die tatsächlich verwendete Magnetresonanzvorrichtung und/oder relevante Komponenten der tatsächlich verwendeten Magnetresonanzvorrichtung eindeutig zu identifizieren. Die tatsächlich verwendete Magnetresonanzvorrichtung ist also die Magnetresonanzvorrichtung, mit der das Verfahren durchgeführt wird.

Die Ist-Geräte-ID wird vorzugsweise vom ersten Teil erfasst. Beispielsweise liest der erste Teil Identifikationsdaten aus einem Speicher, z.B. einen EEPROM-Speicher, der tatsächlich verwendeten Magnetresonanzvorrichtung und/oder relevante Komponenten der tatsächlich verwendeten Magnetresonanzvorrichtung aus. Eine andere Möglichkeit besteht darin, dass während der Herstellung und/oder Installation der Magnetresonanzvorrichtung die Ist-Geräte-ID in einem Speicher der Sicherheitseinheit hinterlegt wird, woraus die Ist-Geräte-ID ausgelesen werden kann. Mit anderen Worten wird die Information über den Anlagentyp vorzugsweise nicht von dem zweiten Teil ermittelt, insbesondere parametriert der zweite Teil nicht aktiv den ersten Teil.

Anhand der Ist-Geräte-ID und dem mindestens einen Konfigurationsparameterdatensatz wird vorzugsweise eine Ist-Prüfsumme gebildet, die mit der Soll-Prüfsumme verglichen werden kann. Sofern Ist-Prüfsumme und Soll-Prüfsumme gleich sind, kann davon ausgegangen werden, dass kein Fehler vorliegt und der übertragene Konfigurationsparameterdatensatz gefahrlos verwendet werden kann, da somit in der Regel sichergestellt ist, dass der übertragene Konfigurationsparameterdatensatz exakt zu dem Ist-Zustand der Magnetresonanzanlage passt. Somit liegt hier eine Ende-zu-Ende-Verschlüsselung des mindestens einen Konfigurationsparameterdatensatzes vor.

Durch die Verifikation des mindestens einen Konfigurationsparameterdatensatz kann abgesichert werden, dass kein Datenübertragungsfehler vorliegt und/oder ein korrekter Konfigurationsparameterdatensatz und/oder abgeschlossener Datensatz vorliegt. Mit dieser Variante des vorgeschlagenen Verfahrens ermöglicht eine effiziente Sicherheitsarchitektur. Insbesondere erlaubt die Ende-zu-Ende-Verschlüsselung eine Verwendung einer Vielzahl an Speicher- und/oder Übertragungsmöglichkeiten.

Eine weitere Ausführungsform des Verfahrens sieht vor, dass die Sicherheitseinheit kontrolliert, ob ein Betriebsfehler, insbesondere ein Softwarefehler und/oder ein Hardwarefehler und/oder ein Energieversorgungsfehler, vorliegt.

Ein Softwarefehler kann beispielsweise einen Absturz eines Programms und/oder eine Applikation umfassen. Ein Hardwarefehler kann beispielsweise einen Defekt eines Hardwaremoduls, wie z.B. eines Prozessors, umfassen. Ein Energieversorgungsfehler liegt beispielsweise vor, wenn die Sicherheitseinheit von einer Stromversorgung unterbrochen wird.

Sofern ein Betriebsfehler vorliegt, kann vorzugsweise ein sicheres Herunterfahren der Magnetresonanzvorrichtung durchgeführt werden, um einen unsicheren Betrieb zu vermeiden.

Eine weitere Ausführungsform des Verfahrens sieht vor, dass ein Funktionstest in einem abgesicherten Betriebsmodus durch die Sicherheitseinheit durchgeführt wird. Der Funktionstest wird vorzugsweise in geeigneten Zeitfenstern durchgeführt, wie z.B. bei jedem Neustart der Sicherheitseinheit.

Bevorzugt wird der Funktionstest in einem abgesicherten Betriebsmodus durch die Sicherheitseinheit durchgeführt. Dieser stellt neben dem eingeschränkten und dem uneingeschränkten Betriebsmodus einen weiteren Betriebsmodus dar, der vorzugsweise niedrigere Grenzwerte aufweist als der eingeschränkter Betriebsmodus und/oder der uneingeschränkter Betriebsmodus.

Bevorzugt wird die Sichereinheitseinheit immer im abgesicherten Betriebsmodus hochgefahren. Vorzugsweise wechselt der Betriebsmodus vom abgesicherten Betriebsmodus in den eingeschränkten Betriebsmodus nur dann, wenn der Funktionstest erfolgreich abgeschlossen wird. Falls der Funktionstest ganz oder teilweise nicht erfolgreich durchgeführt wurde, wird die Magnetresonanzvorrichtung weiterhin im abgesicherten Betriebsmodus betrieben, um etwaige Gefährdungen auszuschließen.

Des Weiteren wird eine Magnetresonanzvorrichtung gemäß Anspruch 15 vorgeschlagen.

Die Vorteile der erfindungsgemäßen Magnetresonanzvorrichtung entsprechen im Wesentlichen den Vorteilen des erfindungsgemäßen Verfahrens zum Betrieb einer Magnetresonanzvorrichtung unter Berücksichtigung von Implantat-Trägern, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Weitere Vorteile, Merkmale und Einzelheiten der Erfindung ergeben sich aus den im Folgenden beschriebenen Ausführungsbeispielen sowie anhand der Zeichnungen. Einander entsprechende Elemente sind in allen Figuren mit den gleichen Bezugszeichen versehen.

Es zeigen:

Fig. 1     eine Ausführungsform einer erfindungsgemäße Magnetresonanzvorrichtung mit einem Sicherheitssystem in einer schematischen Darstellung,

Fig. 2     ein Blockdiagramm einer Ausführungsform eines erfindungsgemäßen Verfahrens,

Fig. 3     ein exemplarischer Ablauf einer Einstellung eines Betriebsmodus gemäß einer ersten Variante des Verfahrens,

Fig. 4     ein exemplarischer Ablauf einer Einstellung eines Betriebsmodus gemäß einer zweiten Variante des Verfahrens,

Fig. 5     eine Darstellung einer möglichen Einstellung eines Betriebsmodus mit Elementen diskreter Logik,

Fig. 6     eine Darstellung einer möglichen Auswertung einer Schaltinformation mit Elementen diskreter Logik,

Fig. 7     ein Blockdiagramm einer ersten Ausführungsform eines erfindungsgemäßen Teilverfahrens zur Bereitstellung mindestens eines Konfigurationsparametersatzes,

Fig. 8 ein Blockdiagramm einer zweiten Ausführungsform eines erfindungsgemäßen Teilverfahrens zur Bereitstellung mindestens eines Konfigurationsparametersatzes,

Fig. 9 ein detailliertes Blockdiagramm einer Variante des zweiten erfindungsgemäßen Teilverfahrens zur Bereitstellung mindestens eines Konfigurationsparametersatzes,

Fig. 10 ein Blockdiagramm eines Ausführungsform eines erfindungsgemäßen Verfahrens mit einem Funktionstest.

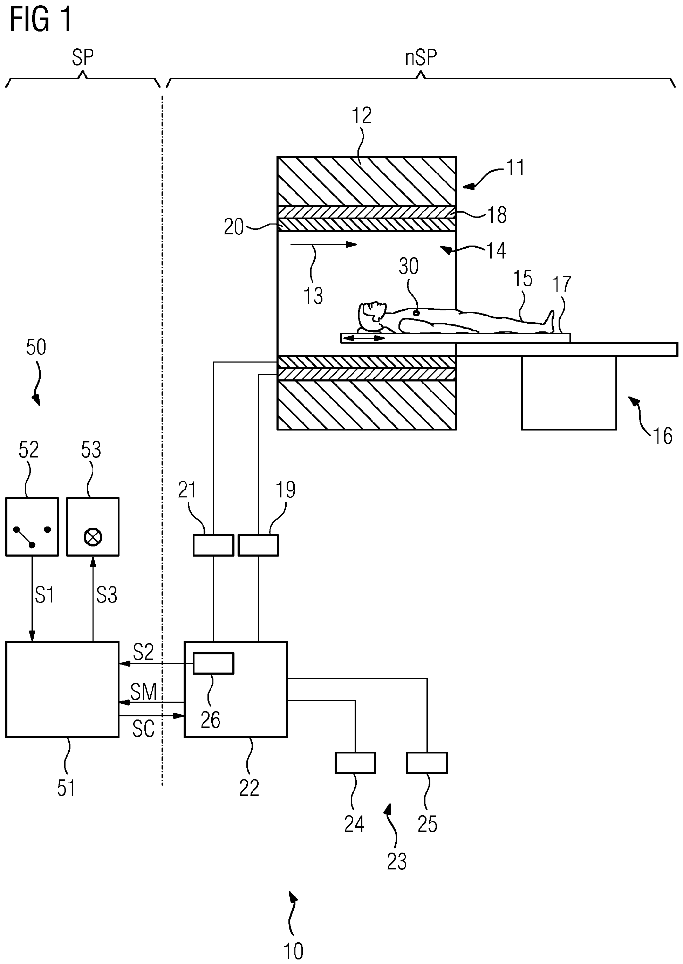

In Fig. 1 ist eine Magnetresonanzvorrichtung 10 schematisch dargestellt. Die Magnetresonanzvorrichtung 10 umfasst eine Magneteinheit 11, die einen, beispielsweise supraleitenden, Hauptmagneten 12 zu einem Erzeugen eines starken und insbesondere zeitlich konstanten Hauptmagnetfelds 13 aufweist. Zudem umfasst die Magnetresonanzvorrichtung 10 einen Patientenaufnahmebereich 14 zu einer Aufnahme eines Patienten 15. Der Patient kann ein Implantat-Träger sein, so dass sich beispielsweise ein Herzschrittmacher 30 in seinem Körper befindet. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der Magnetresonanzvorrichtung 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Gradientenfeldern auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Magneteinheit 11 umfasst weiterhin eine Hochfrequenzantenneneinheit 20, welche im vorliegenden Ausführungsbeispiel als fest in die Magnetresonanzvorrichtung 10 integrierte Körperspule ausgebildet ist. Die Hochfrequenzantenneneinheit 20 ist zu einer Anregung von Atomkernen, die sich in dem von dem Hauptmagneten 12 erzeugten Hauptmagnetfeld 13 einstellt, ausgelegt. Die Hochfrequenzantenneneinheit 20 wird von einer Hochfrequenzantennensteuereinheit 21 der Magnetresonanzvorrichtung 10 gesteuert und erzeugt Hochfrequenzfelder in einem Untersuchungsraum, der im Wesentlichen von einem Patientenaufnahmebereich 14 der Magnetresonanzvorrichtung 10 gebildet ist. Die Hochfrequenzantenneneinheit 20 ist weiterhin zum Empfang von Magnetresonanzsignalen ausgebildet.

Zu einer Steuerung des Hauptmagneten 12, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung 10, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung von medizinischen Bilddaten, die während der Magnetresonanzuntersuchung erfasst werden. Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzbilder können auf einem Monitor 24 der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können. Die Systemsteuereinheit weist zudem eine Patientenregistrierungseinheit 26 auf.

Die bisher erläuterten Komponenten der Magnetresonanzvorrichtung 10 werden von einem zweiten Teil nSP umfasst, während ein erster Teil SP der Magnetresonanzvorrichtung 10 ein Sicherheitssystem 50 umfasst, das eine Sicherheitseinheit 51, eine Schalteinheit 52 und eine Anzeigeeinheit 53 aufweist. Die Sicherheitseinheit 51 steht in Verbindung mit der Systemsteuereinheit 22 und ist ausgebildet, etwa bei einer Untersuchung eines Implantat-Trägers, die Magnetresonanzvorrichtung 10 in einem eingeschränkten Betriebsmodus auf Einhaltung Implantats-konformer Grenzwerte zu kontrollieren.

Der erste Teil SP der Magnetresonanzvorrichtung erfüllt hier höhere Sicherheitsanforderungen als der zweite Teil nSP. Dadurch erfolgt Kontrolle auf Einhaltung Implantats-konformer Grenzwerte vorteilhafterweise besonders sicher. Erfindungsgemäß weist der erste Teil SP Programmmittel gemäß einer ersten Sicherheitskategorie auf und der zweite Teil nSP Programmmittel gemäß einer zweiten Sicherheitskategorie, wobei die erste Sicherheitskategorie höhere Sicherheitsanforderungen als die zweite Sicherheitskategorie aufweist. Der erste Teil SP wird getrennt vom zweiten Teil nSP betrieben, so dass durch den zweiten Teil nSP keine sicherheitskritische Wechselwirkung mit dem ersten Teil SP möglich ist.

Die Implantats-konformen Grenzwerte werden beispielsweise von einer Norm vorgegeben, wie z.B. der Norm IEC 60601-2-33. Dort ist ein Betriebsmodus gemäß FPO:B angegeben, der vorsieht, dass insbesondere für die Werte $B_1^+{}_{peak}$, $B_1^+{}_{rms}$, $(|dB/dt|_{peak})_{FPO}$ und $(dB/dt|_{rms})_{FPO}$ gewisse Grenzwerte einzuhalten sind. Peak-Werte, wie z.B. $B_1^+{}_{peak}$

und/oder $(|dB/dt|_{peak})_{FPO}$, werden vorzugsweise permanent überwacht. Effektiv-Werte, wie z.B. $B_1^+{}_{rms}$ und/oder $(|dB/dt|_{rms})_{FPO}$ werden vorzugsweise über einen ersten Zeitabstand, von z.B. 10 Sekunden, gemittelt und/oder im Takt eines zweiten Zeitabstands, von z.B. 1 Sekunden, überprüft. Damit soll sichergestellt werden, dass durch eine Magnetresonanzuntersuchung von Patienten, die ein Implantat gemäß FPO:B in sich tragen, keine Gefährdung für den Patienten ausgeht. Der eingeschränkte Betriebsmodus erlaubt in diesem Fall vorteilhafterweise also nur einen Betrieb der Magnetresonanzvorrichtung, solange die Grenzwerte gemäß FPO:B eingehalten werden.

Zur Kontrolle auf Einhaltung der Implantats-konformen Grenzwerte werden der Sicherheitseinheit während der Untersuchung Sicherheitsmessdaten SM bereitgestellt, wie beispielsweise Daten über breitbandige und/oder schmalbandige hochfrequente Anregungssignale der Hochfrequenzantenneneinheit 20 und/oder Daten über Gradientenströme der Gradientenspuleneinheit 18. Vorzugsweise werden diese Daten zumindest teilweise verifiziert, um sicherzustellen, dass die Daten korrekt erfasst und übertragen wurden. Dafür sind verschiedene Mechanismen denkbar, wie beispielsweise ein redundanter Messdatenstrom, der, insbesondere je nach Risiko von Ausfall und/oder Verfälschung, permanent oder zyklisch gegeneinander geprüft wird.

Sollte eine Überschreitung eines oder mehrerer Implantats-konformer Grenzwerte festgestellt werden, übermittelt die Sicherheitseinheit 51 ein Kontrollsignal SC an die Systemsteuereinheit 22, welcher zu einer sicheren Abschaltung der Magnetresonanzvorrichtung 10 führt.

Zudem kontrolliert die Sicherheitseinheit 51, ob ein Betriebsfehler vorliegt. Falls dies der Fall ist, übermittelt die Sicherheitseinheit 51 ein Kontrollsignal SC an die Systemsteuereinheit 22, wodurch eine sichere Abschaltung der Magnetresonanzvorrichtung 10 ausgelöst wird. Ein solcher Betriebsfehler kann beispielsweise einen Softwarefehler und/oder einen Hardwarefehler und/oder einen Energieversorgungsfehler umfassen.

Die Schalteinheit 52 ist ausgebildet, eine Schaltinformation S1 an die Sicherheitseinheit 51 zu übermitteln. Die Schalteinheit 52 kann beispielsweise als ein mechanischer Schalter oder mechanischer Taster ausgeführt werden. Es ist aber auch denkbar, dass die Schalteinheit 52 in Form einer elektronischen Benutzerschnittstelle umgesetzt wird, bei der eine Bedienung des Schalters über ein Eingabegerät, wie z.B. eine Computermaus und/oder eine Tastatur erfolgt. Das Eingabegerät kann eine von der Benutzerschnittstelle 23 eigenständige Vorrichtung sein, um hier eine Verflechtung des ersten Teils SP und zweiten Teils nSP zu vermeiden. Es ist aber auch denkbar, dass die Schalteinheit 52 aus dem zweiten Teil nSP, beispielsweise von der Benutzerschnittstelle 23, gesteuert wird. In diesem Fall werden vorzugsweise externe Maßnahmen vorgenommen, wie z.B. eine Kontrolle des Betriebsmodus durch das Bedienpersonal, um eine ausreichende Sicherheit, insbesondere gemäß der Sicherheitsklasse A, zu gewährleisten.

Ein Bedienpersonal kann mit Hilfe der Schalteinheit 52 einstellen, ob ein Betrieb der Magnetresonanzvorrichtung 10 im eingeschränkten Betriebsmodus, insbesondere gemäß FPO:B, erwünscht ist oder nicht. Im ersteren Fall, also falls der eingeschränkte Betriebsmodus aktiv sein soll, wird der Sicherheitseinheit 51 eine aktive Schaltinformation S1 bereitgestellt. Im letzteren Fall, also falls der eingeschränkte Betriebsmodus nicht aktiv sein soll, sondern stattdessen beispielsweise ein uneingeschränkter Betriebsmodus, wird der Sicherheitseinheit 51 eine passive Schaltinformation S1 bereitgestellt.

Findet ein Patientenwechsel statt, gibt die Patientenregistrierungseinheit 26 eine Patientenregistrierungsinformation S2 an die Sichereinheitseinheit 51 weiter und veranlasst die Sicherheitseinheit 51, die Schaltinformation S1 auszuwerten. Die Sicherheitseinheit 51 stellt also anhand der bereitgestellten Informationen, also der Patientenregistrierungsinformation S2 und der Schaltinformation S1, einen bestimmten Betriebszustand ein. Außerdem wird der eingestellte Betriebszustand durch die Anzeigeeinheit 53, z.B. eine Lichtdiode (engl. light emitting diode, LED), angezeigt. Zu diesem Zweck wird ein Anzeigesignal S3 von der Sicherheitseinheit 51 an die Anzeigeeinheit 53 übertragen. So kann das Bedienpersonal kontrollieren, ob die Anzeige dem Patienten entspricht, z.B. eine grüne LED-Anzeige bei einem Implantat aufweisenden Patienten und eine rote LED-Anzeige bei einem Implantat freien Patienten.

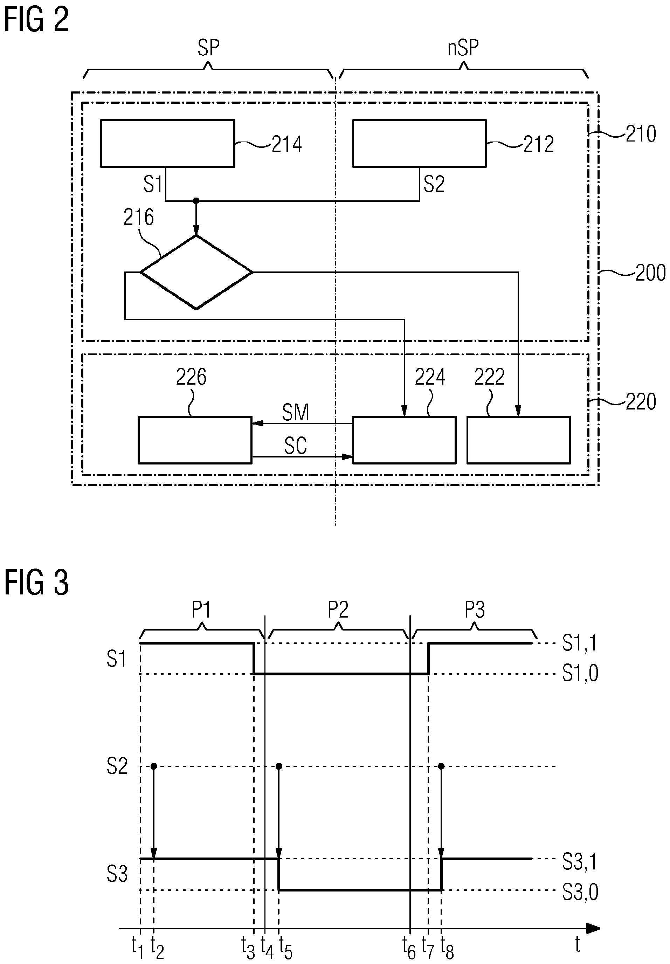

In Fig. 2 ist ein Schema eines möglichen Verfahrens zum Betrieb der Magnetresonanzvorrichtung 10 unter Berücksichtigung von Implantat-Trägern mittels der Sicherheitseinheit 51 dargestellt. Eine Messung 200 umfasst dabei eine Messvorbereitung 210 und eine Messdurchführung 220. In einem Schritt 214 der Messvorbereitung 210 erfolgt eine Bereitstellung einer Schaltinformation S1 durch die Schalteinheit 52 an die Sicherheitseinheit 51. Schalteinheit 52 und Sicherheitseinheit 51 sind Bestandteile des ersten Teils SP der Magnetresonanzvorrichtung 10, so dass auch dieser Teil des Verfahrens im ersten Teil SP erfolgt.

Aus dem zweiten Teil nSP wird in Schritt 212 eine Patientenregistrierungsinformation S2 von der Patientenregistrierungseinheit 26 an die Sicherheitseinheit 51 übertragen. Dies erfolgt vorteilhafterweise dann, wenn an einem neuen Patienten 15 eine Messdurchführung ansteht. Auf diese Nachricht und/oder Trigger, wird in Schritt 216 ein Betriebsmodus ermittelt, welcher in der Messdurchführung 200 angewandt werden soll. Bei Ermittlung eines eingeschränkten Betriebsmodus erfolgt in Schritt 224 die Messdurchführung demzufolge im eingeschränkten Betriebsmodus. Dabei wird in Schritt 226 die Messung durch die Sicherheitseinheit 51 kontrolliert. Dazu werden der Sicherheitseinheit 51 Sicherheitsmessdaten SM übertragen.

Mit Hilfe von Kontrollsignalen SC kann die Sicherheitseinheit 51 die Messung 224 im eingeschränkten Betriebsmodus beeinflussen, insbesondere bei Überschreitung eines Implantats-konformen Grenzwertes abbrechen. Bei Ermittlung eines uneingeschränkten Betriebsmodus in Schritt 216 erfolgt in Schritt 222 die Messdurchführung demzufolge im uneingeschränkten Betriebsmodus ohne Kontrolle durch die Sicherheitseinheit 51.

In den Fig. 3 bis 6 sind eine Ermittlung und eine daraus resultierende Einstellung des Betriebsmodus in Schritt 216 detailliert illustriert. So zeigt Fig. 3 einen Verlauf entlang der Zeit t gemäß einer ersten Variante des Verfahrens. Die Sicherheitseinheit 51 empfängt eine Schaltinformation S1, z.B. in Form eines, insbesondere elektronischen und/oder elektrischen, Signals, aus mehreren möglichen Schaltinformationen. Dabei sind hier zwei Schaltinformationen möglich, nämlich eine aktive Schaltinformation S1,1 und eine passive Schaltinformation S1,0. Diese zwei Schaltinformationen entsprechen beispielsweise zwei Schaltzuständen der Schalteinheit 52, die durch das Bedienpersonal einstellbar sind. Ferner empfängt die Sicherheitseinheit 51 zu verschiedenen Zeitpunkten $t_2$, $t_5$, $t_8$ von der Patientenregistrierungseinheit 26 eine Patientenregistrierungsinformation S2, z.B. in Form eines, insbesondere elektronischen und/oder elektrischen, Signals, welches als Trigger für die Auswertung der Schaltinformation dient. Die Sicherheitseinheit 51 stellt abhängig von der bereitgestellten Schaltinformation S1 und von der bereitgestellten Patientenregistrierungsinformation S2 einen Betriebsmodus aus mehreren möglichen Betriebsmodi ein. Der eingestellte Betriebsmodus wird durch die Anzeigeeinheit 53 dargestellt, z.B. durch Einschalten einer Lichtdiode, so dass hier ein bestimmter Betriebsmodus einem bestimmten Anzeigesignal S3 entspricht. Hier sind zwei mögliche Betriebsmodi dargestellt, nämlich der eingeschränkte Betriebsmodus S3,1 und der uneingeschränkte Betriebsmodus S3,0.

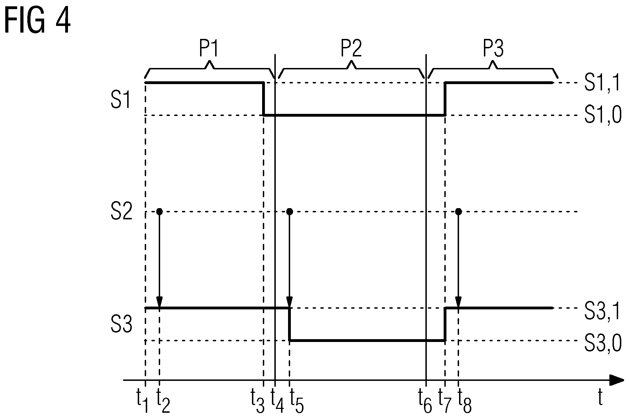

In dem in Fig. 3 und 4 dargestellten Beispielen werden drei Patienten P1, P2, P3 mit Hilfe der Magnetresonanzvorrichtung 10, wobei die Patienten P1 und P3 Implantat-Träger seien. Für die Untersuchung des ersten Patienten P1 ist ein erster Zeitraum zwischen den Zeitpunkten $t_1$ und $t_4$ vorgesehen, für die Untersuchung des zweiten Patienten P2 ist ein zweiter Zeitraum den Zeitpunkten $t_4$ und $t_6$ vorgesehen und der dritte Patient P3 soll nach dem Zeitpunkt $t_6$ untersucht werden.

Der initiale Betriebsmodus ist der eingeschränkte Betriebsmodus S3,1. Zum Zeitpunkt $t_1$ erfolgt ein Wechsel des Schaltzustands von S1,0 auf S1,1. Dies erfolgt beispielsweise durch das Bedienpersonal der Magnetresonanzvorrichtung 10 in der Kenntnis, dass nun eine Untersuchung eines Implantat-Trägers ansteht. Im weiteren Verlauf der Untersuchung, beispielsweise während einer Vorbereitungsphase, in der eine Registrierung des Patienten P1 erfolgt, sendet die Patientenregistrierungseinheit 52 zum Zeitpunkt $t_2$ eine Patientenregistrierungsinformation S2 zur Sicherheitseinheit 51. Die Schaltinformation S1 wird daraufhin ausgewertet, so dass der Betriebsmodus darauf weiterhin im eingeschränkten Betriebsmodus S3,1 verbleibt, da zu diesem Zeitpunkt die aktive Schaltinformation S1,1 bereitgestellt wird.

Zum Zeitpunkt $t_3$ wird der Schaltzustand der Schalteinheit auf S1,0 geändert, beispielsweise, weil die Aufnahme der Magnetresonanzdaten beendet wurde, der erste Patient P1 sich nicht mehr im Patientenaufnahmebereich 14 befindet und das Bedienpersonal davon ausgeht, dass der nächste Patient kein Implantat-Träger ist. Zum Zeitpunkt $t_3$ wird jedoch der Betriebszustand S3 (noch) nicht geändert. Erst wenn zum Zeitpunkt $t_5$ für den zweiten Patienten P2 eine Patientenregistrierungsinformation S2 von der Sicherheitseinheit 51 empfangen wird, erfolgt ein Wechsel des Betriebsmodus. Der uneingeschränkte Betriebsmodus S3,0 wird also nur dann eingestellt, wenn zum Zeitpunkt der Bereitstellung der Patientenregistrierungsinformation S2 die breitgestellte Schaltinformation S1 die passive Schaltinformation S1,0 ist.

Eine Bereitstellung einer Patientenregistrierungsinformation S2 allein löst keine Deaktivierung des eingeschränkten Betriebszustand aus, sondern diese erfolgt nur, wenn gleichzeitig die aktuelle Schaltinformation S1 eine passive Schaltinformation S1,0 ist. Daher ist die Bereitstellung der Patientenregistrierungsinformation S2 nicht sicherheitskritisch.

Zum Zeitpunkt $t_7$ wird eine aktive Schaltinformation S1,1 eingestellt. Gemäß der in Fig. 3 dargestellten Variante wird diese Schaltinformation erst zum Zeitpunkt $t_8$ ausgelöst durch eine Patientenregistrierungsinformation S2 ausgewertet. Aus der Auswertung resultiert ein Wechsel in den eingeschränkten Betriebsmodus S3,1.

Dagegen erfolgt gemäß der in Fig. 4 dargestellten Variante der Wechsel in den eingeschränkten Betriebsmodus S3,1 bereits zum Zeitpunkt $t_7$ zeitgleich mit dem Wechsel der Schaltinformation von S1,0 auf S1,1. Eine Aktivierung des eingeschränkten Betriebsmodus S3,1 bedarf hier also keiner Triggerung durch eine Patientenregistrierungsinformation S2.

Die Generierung und/oder Übermittlung der Schaltinformation S1 und/oder das Anzeigesignal S3 wird vorzugsweise als Hardwarelösung umgesetzt, so dass diese besonders sicher durchgeführt wird und insbesondere die Sicherheitsklassen C der Norm IEC 62304 erfüllen.

Das vorgeschlagene Sicherheitskonzept erlaubt es beispielsweise, die Generierung der Patientenregistrierungsinformation S2 mittels einer Software gemäß der Sicherheitsklasse B auszuführen. Falls dabei ein Fehler auftritt, so dass der Sicherheitseinheit 51 keine Patienteninformation S2 bereitgestellt wird, lassen sich insbesondere folgende Fälle unterscheiden:

A) Falls der eingeschränkte Betriebsmodus S3,1 gegeben ist und die passive Schaltinformation S1,0 eingestellt wird, so verbleibt die Magnetresonanzvorrichtung 10 weiterhin im eingeschränkten Betriebsmodus S3,1, solange keine Patienteninformation S2 bereitgestellt wird. Gegebenenfalls erkennt das Bedienpersonal die Fehlfunktion mittels der Anzeigeeinheit 53 und wiederholt die Registrierung solange, bis der der gewünschte uneingeschränkte Betriebsmodus S3,0 aktiviert ist und angezeigt wird.

B) Falls der uneingeschränkte Betriebsmodus S3,0 gegeben ist und die aktive Schaltinformation S1,1 eingestellt wird, lassen sich zwei Varianten unterscheiden: Gemäß Fig. 3 verbleibt die Magnetresonanzvorrichtung 10 weiterhin im uneingeschränkten Betriebsmodus S3,0, solange keine Patienteninformation S2 bereitgestellt wird. Gegebenenfalls erkennt das Bedienpersonal die Fehlfunktion mittels der Anzeigeeinheit 53 und wiederholt die Registrierung solange, bis der der gewünschte eingeschränkte Betriebsmodus S3,1 aktiviert ist und angezeigt wird. Gemäß Fig. 4 wird sofort der eingeschränkte Betriebsmodus S3,1 aktiviert, sobald die aktive Schaltinformation S1,1 eingestellt wird.

Das Anzeigezustand und/oder der Betriebsmodus S3 zu einem Zeitpunkt t in Abhängigkeit der Schaltinformation S1 und des Patientenregistrierungsinformation S2 zum Zeitpunkt t sowie des Betriebsmodus S3 zu einem früheren Zeitpunkt t-1 kann mit folgender logischen Gleichung beschrieben werden, die zum Zeitpunkt $t_2$, $t_5$, $t_8$ der Bereitstellung der Patienteninformation S2 gültig ist:

$$S3(t) = (S1(t) \vee S3(t-1)) \wedge \overline{(\overline{S1(t)} \wedge S2(t))}$$

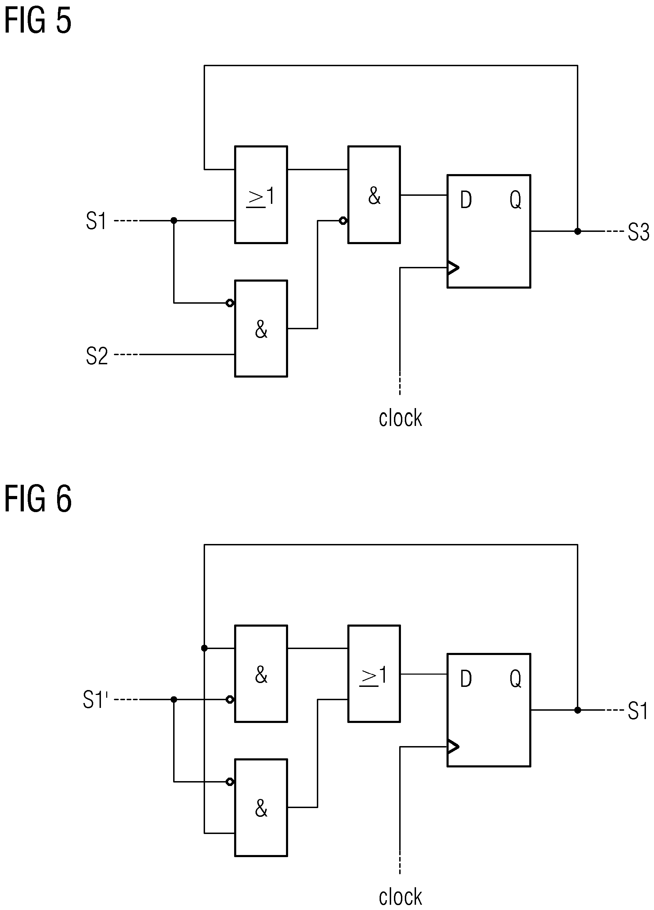

Diese Gleichung kann in die Sicherheitseinheit 51 beispielsweise mittels eines Softwareprogramm und/oder einer diskreten Logik durch Verwendung einer Schaltung, wie sie in Fig. 5 dargestellt ist, implementiert werden.
Mit diesem Design wird eine Deaktivierung des eingeschränkten Betriebsmodus S3,1 allein mittels der Schalteinheit 52 und der Sicherheitseinheit 51 kontrolliert, welche als Bestandteile des ersten Teils SR besonders sicherheitsrelevant sind. Die Bereitstellung der Patientenregistrierungsinformation S2 dient hier vor allem als Trigger, wann die Schaltinformation S1 auszuwerten ist, aber deaktiviert selbst den eingeschränkten Betriebsmodus S3,1 nicht.
In Fig. 6 ist eine Variante dieser Konfiguration gezeigt, welche anstelle eines Schalters einen Taster verwendet. Diese hat den Vorteil, dass nur die Anzeigeeinheit den momentanen Betriebsmodus anzeigt und das Bedienpersonal nicht durch eine sichtbare mechanische Schaltposition eines Schalters möglicherweise irregeführt wird. Der Taster erzeugt ein Signal S1', welcher den Zustand eines Latch-Registers umschaltet, wobei die Ausgabe dieses Latch-Registers als Schaltinformation S1 verwendet wird. Mit der folgenden Gleichung, die zum Zeitpunkt $t_2$, $t_5$, $t_8$ der Bereitstellung der Patienteninformation S2 gültig ist, lässt sich analog zur obigen Gleichung sowie zu Fig. 3 beschreiben mit:

$$S3(t) = \left(\overline{S1'(t)} \wedge S1(t-1)\right) \vee \left(S1'(t) \wedge \overline{S1(k-1)}\right)$$

Anhand von Fig. 2 wurde bereits die Kontrolle auf Einhaltung Implantats-konformer Grenzwerte in Schritt 226 erläutert. Zu diesem Zweck werden der Sicherheitseinheit 51 neben den bereits erwähnten Sicherheitsmessdaten SM vorzugsweise mindestens ein Konfigurationsparameterdatensatz bereitgestellt, der beispielsweise Angaben zu einer Empfindlichkeit von Gradientenspulen und/oder Skalierungsfaktoren wie z.B. Stärke eines resultierendes $B_1^+$-Feld pro Volt einer angelegten Spannung, umfasst. Mit Hilfe solcher Angaben kann eine Auswertung der Sicherheitsmessdaten SM erleichtert werden.

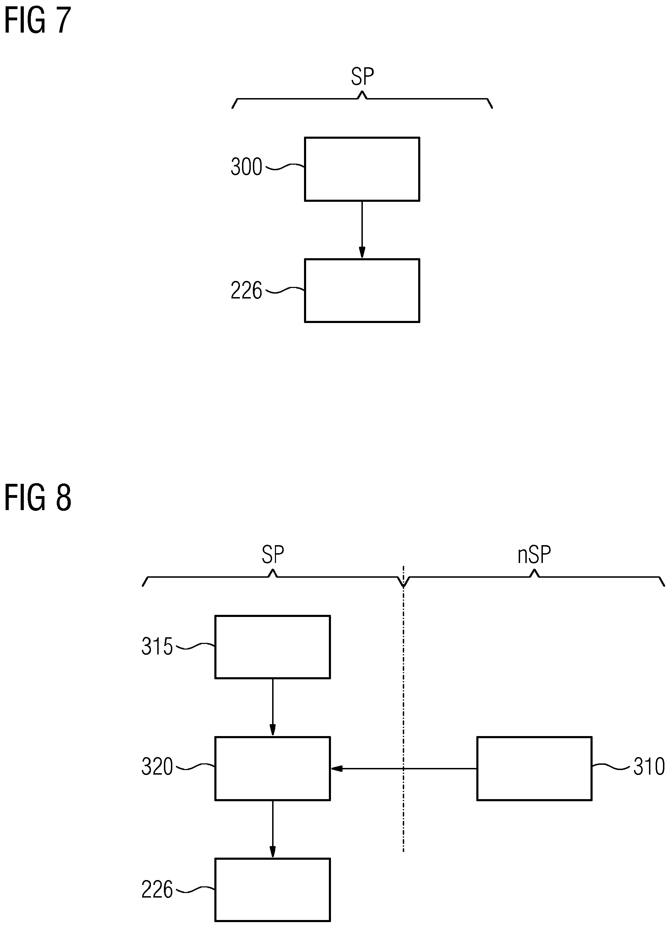

In Fig. 7 ist eine mögliche Ausführungsform für eine Bereitstellung des mindestens einen Konfigurationsparametersatzes dargestellt, anhand dessen im Schritt 226 die Kontrolle auf Einhaltung der Implantats-konformen Grenzwerte erfolgt. Dabei ist der mindestens eine Konfigurationsparameterdatensatz im ersten Teil SP hinterlegt und wird in einem Schritt 300 bereitgestellt. Somit kann eine Übertragung des mindestens einen Konfigurationsparametersatzes von außerhalb des ersten Teils SP vermieden werden.
Dabei kann ein fixer Konfigurationsparameterdatensatz verwendet werden, der beispielsweise innerhalb der Sicherheitseinheit 51 abgespeichert ist. Gemäß einer möglichen Variante ist der fixe Konfigurationsparameterdatensatz derart ausgestaltet, dass er konservativ eine Einhüllende über eine Vielzahl von Gradientenspuleneinheiten und/oder Hochfrequenzantenneneinheiten bildet, für die die Sicherheitseinheit 51 anwendbar sein soll. Damit kann eine einzelne Sicherheitseinheit 51 zwar in einer Vielzahl an unterschiedlich ausgebildeten Magnetresonanzvorrichtungen eingesetzt werden, jedoch wird dadurch die maximal mögliche Leistungsfähigkeit der Magnetresonanzvorrichtungen möglicherweise nicht ausgenutzt.
Gemäß einer hier nicht näher dargestellten Variante umfasst der zumindest eine Konfigurationsparameterdatensatz mehrere Konfigurationsparameterdatensätze, wobei aus den mehreren Konfigurationsparameterdatensätzen ein Konfigurationsparameterdatensatz abhängig von Parametern der Magnetresonanzvorrichtung 10 ausgewählt wird.

Hierbei können insbesondere zwei Möglichkeiten unterschieden werden: A) Die für den Anlagentyp gültige Konfigurationsparameterdatensatz wird bei der Herstellung und/oder Installation vorzugsweise durch mehrere Personen fest eingestellt (Vier-Augen-Prinzip). B) Der Anlagentyp wird, insbesondere automatisch mittels einer Hardware-Erkennung, erkannt und an die Sicherheitseinheit 51 übertragen.

Fig. 8 zeigt eine weitere Möglichkeit zur Bereitstellung des mindestens einen Konfigurationsparameterdatensatzes. In einem Schritt 310 wird insbesondere ein im zweiten Teil nSP hinterlegter Konfigurationsparameterdatensatz der Sicherheitseinheit 51 bereitgestellt, welcher in einem Schritt 320 anhand einer Prüfsumme verifiziert wird. In Schritt 315 wird eine Ist-Geräte-ID 408 bereitgestellt, die durch den ersten Teil SP beispielsweise mittels Hardware-Erkennung ermittelt wird und/oder durch Programmmittel der Sicherheitseinheit 51 aus einem Speicher des ersten Teils SP ausgelesen wird, in welchen die Ist-Geräte-ID z.B. im Rahmen der Herstellung und/oder Installation der Magnetresonanzvorrichtung 10 hinterlegt wird. Sofern die Verifikation erfolgreich ist, erfolgt im Schritt 226 die Kontrolle auf Einhaltung der Implantats-konformen Grenzwerte unter Verwendung des Konfigurationsparameterdatensatzes.

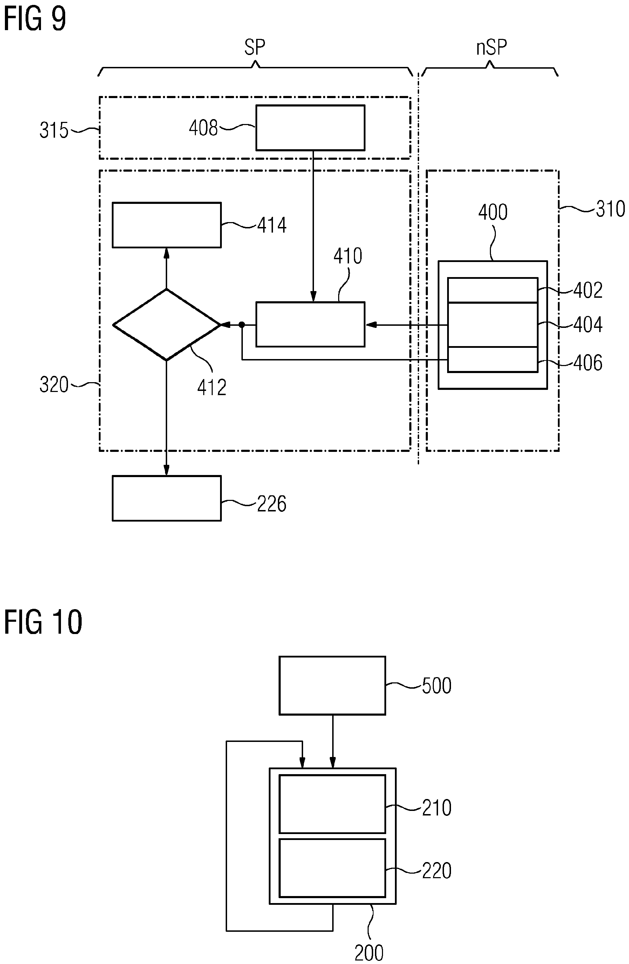

Eine detailliertere Ausführungsform wird durch Fig. 9 dargestellt. In Schritt 310 wird ein abgeschlossener Datensatz 400 bereitgestellt, der eine Soll-Geräte-ID 402, einen Konfigurationsparameterdatensatz 404 und eine Soll-Prüfsumme 406 umfasst.

Die Ist-Geräte-ID vorzugsweise ausgebildet, die tatsächlich verwendete Magnetresonanzvorrichtung 10 und/oder relevante Komponenten der tatsächlich verwendeten Magnetresonanzvorrichtung 10, wie beispielsweise die Hochfrequenzantenneneinheit 20 und/oder die Gradientenspuleneinheit 18, eindeutig zu identifizieren.

Die Soll-Prüfsumme 406 wird anhand der Soll-Geräte-ID 402 und dem Konfigurationsparameterdatensatz 404 erzeugt. Die Soll-Geräte-ID 402 ist charakteristisch für einen Typ der Magnetresonanzvorrichtung 10 und/oder relevanter Komponenten der Magnetresonanzvorrichtung 10, wie beispielsweise einen Typ der Hochfrequenzantenneneinheit 20 und/oder einen Typ der Gradientenspuleneinheit 18.

Im Schritt 410 wird im ersten Teil SP anhand der Ist-Geräte-ID und dem Konfigurationsparameterdatensatz 404 eine Ist-Prüfsumme erzeugt, welche im Schritt 412 mit der Soll-Prüfsumme 406 verglichen wird. Ergibt diese Überprüfung, dass die Ist-Prüfsumme und die Soll-Prüfsumme 406 gleich sind, so erfolgt im Schritt 226 die Kontrolle auf Einhaltung der Implantats-konformen Grenzwerte unter Verwendung des Konfigurationsparameterdatensatzes 404. Anderenfalls wird der Konfigurationsparameterdatensatzes 404 nicht verwendet und die Verifikation des Konfigurationsparameterdatensatzes 404 in Schritt 414 beendet.

Der abgeschlossene Datensatz 400 kann beispielsweise im Rahmen der Entwicklung und/oder Herstellung der Magnetresonanzvorrichtung 10 erzeugt werden und außerhalb der ersten Einheit, beispielsweise in der Systemsteuereinheit 22 und/oder zusammen mit anderen Parametrierungen der Magnetresonanzvorrichtung, abgespeichert werden. Die hier dargestellte Ende-zu-Ende-Verschlüsselung erlaubt einen Rückgriff auf konventionelle Speicher- und Übertragungstechniken und/oder Techniken, deren Entwicklung oft weniger strengen Restriktionen unterliegen.

In Fig. 10 ist ein erweitertes Verfahren dargestellt, mit einem Funktionstest in Schritt 500. Der Funktionstest wird vorzugsweise in einem abgesicherten Betriebsmodus durch die Sicherheitseinheit 51 durchgeführt. Der abgesicherte Betriebsmodus weist üblicherweise niedrigere Grenzwerte auf als der eingeschränkte Betriebsmodus und/oder der uneingeschränkter Betriebsmodus. Der Betriebsmodus wechselt vom abgesicherten Betriebsmodus in den eingeschränkten Betriebsmodus nur dann, wenn der Funktionstest erfolgreich abgeschlossen wird.

Der Schritt 500 wird in zu bestimmenden zeitlichen Abständen durchgeführt, beispielsweise stündlich, täglich oder wöchentlich und/oder in geeigneten Zeitfenstern durchgeführt, wie z.B. bei jedem Neustart der Sicherheitseinheit. Der Funktionstest muss also nicht vor jeder Untersuchung durchgeführt werden, sondern es können zwischen zwei Funktionstests mehrere Messungen 200 durchgeführt werden.

Es wird abschließend noch einmal darauf hingewiesen, dass es sich bei den vorhergehend detailliert beschriebenen Verfahren sowie bei der dargestellten Magnetresonanzvorrichtung lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Modul" nicht aus, dass die betreffenden Komponenten aus mehreren zusammenwirkenden Teil-Komponenten bestehen, die gegebenenfalls auch räumlich verteilt sein können.

**Patentansprüche**

1. Verfahren zum Betrieb einer Magnetresonanzvorrichtung (10) unter Berücksichtigung von Implantat-Trägern (15) mittels einer Sicherheitseinheit (51),
   wobei die Magnetresonanzvorrichtung (10) einen ersten Teil (SP) und einen zweiten Teil (nSP) umfasst,
   wobei der erste Teil (SP) getrennt von dem zweiten Teil (nSP) betrieben wird und die Sicherheitseinheit (51) umfasst,
   wobei der zweite Teil (nSP) eine Systemsteuereinheit (22) zur Steuerung der Magnetresonanzvorrichtung (10) aufweist,

wobei die Sicherheitseinheit (51) ausgebildet ist, Kontrollsignale (SC) an die Systemsteuereinheit (22) des zweiten Teils (nSP) zu senden,

wobei bei einer Untersuchung eines Implantat-Trägers die Sicherheitseinheit (51) die Magnetresonanzvorrichtung (10) in einem eingeschränkten Betriebsmodus auf Einhaltung Implantats-konformer Grenzwerte kontrolliert, **dadurch gekennzeichnet, dass** der erste Teil (SP) mit Software, die Sicherheitsanforderungen gemäß einer ersten Sicherheitskategorie erfüllt, betrieben wird,

wobei der zweite Teil (nSP) mit Software, die Sicherheitsanforderungen gemäß einer zweiten Sicherheitskategorie erfüllt, betrieben wird,

wobei die erste Sicherheitskategorie höhere Sicherheitsanforderungen als die zweite Sicherheitskategorie aufweist.

2. Verfahren nach Anspruch 1,

wobei der erste Teil (SP) eine Schalteinheit (52) umfasst, wobei der Sicherheitseinheit (51) durch die Schalteinheit (52) eine Schaltinformation (S1) aus mehreren möglichen Schaltinformationen bereitgestellt wird,

wobei durch die Sicherheitseinheit (51) abhängig von der bereitgestellten Schaltinformation (S1) ein Betriebsmodus (S3) aus mehreren möglichen Betriebsmodi eingestellt wird.

3. Verfahren nach Anspruch 2,

wobei der zweite Teil (nSP) eine Patientenregistrierungseinheit (26) umfasst,

wobei der Sicherheitseinheit (51) durch die Patientenregistrierungseinheit (26) eine Patientenregistrierungsinformation (S2) bereitgestellt wird,

wobei ein Wechsel des Betriebsmodus (S3) zumindest teilweise durch die bereitgestellte Patientenregistrierungsinformation (S2) ausgelöst wird.

4. Verfahren nach Anspruch 3,

wobei die mehreren möglichen Betriebsmodi (S3) einen uneingeschränkten Betriebsmodus (S3,0) und den eingeschränkten Betriebsmodus (S3,1) umfassen,

wobei die mehreren möglichen Schaltinformationen (S1) eine aktive Schaltinformation (S1,1) und eine passive Schaltinformation (S1,0) umfassen,

wobei der uneingeschränkte Betriebsmodus (S3,0) eingestellt wird, wenn zum Zeitpunkt der Bereitstellung der Patientenregistrierungsinformation (S2) die bereitgestellte Schaltinformation (S1) die passive Schaltinformation (S1,0) ist.

5. Verfahren nach Anspruch 4,

wobei der eingeschränkte Betriebsmodus (S3,1) eingestellt wird, wenn zum Zeitpunkt der Bereitstellung der Patientenregistrierungsinformation (S2) die bereitgestellte Schaltinformation (S1) die aktive Schaltinformation (S1,1) ist oder wenn die bereitgestellte Schaltinformation (S1) die aktive Schaltinformation (S1,1) ist.

6. Verfahren nach einem der Ansprüche 2 bis 5,

wobei der erste Teil (SP) eine Anzeigeeinheit (53) umfasst, die den Betriebsmodus (S3) anzeigt.

7. Verfahren nach einem der vorangehenden Ansprüche,

wobei der Sicherheitseinheit (51) während der Untersuchung Sicherheitsmessdaten (SM) bereitgestellt werden, anhand derer die Kontrolle auf Einhaltung der Implantats-konformen Grenzwerte erfolgt,

wobei die bereitgestellten Sicherheitsmessdaten (SM) zumindest teilweise verifiziert werden.

8. Verfahren nach einem der vorangehenden Ansprüche,

wobei der Sicherheitseinheit (51) mindestens ein Konfigurationsparameterdatensatz (404) bereitgestellt wird, anhand dessen die Kontrolle auf Einhaltung der Implantats-konformen Grenzwerte erfolgt.

9. Verfahren nach Anspruch 8,

wobei der zumindest eine Konfigurationsparameterdatensatz (404) mehrere Konfigurationsparameterdatensätze umfasst, wobei aus den mehreren Konfigurationsparameterdatensätzen ein Konfigurationsparameterdatensatz (404) abhängig von Parametern der Magnetresonanzvorrichtung (10) ausgewählt wird.

10. Verfahren nach einem der Ansprüche 8 oder 9,

wobei der mindestens eine Konfigurationsparameterdatensatz (404) zumindest teilweise von dem zweiten Teil (nSP) in den ersten Teil (SP) übertragen wird,

wobei der mindestens eine bereitgestellte Konfigurationsparameterdatensatz (404) anhand einer Prüfsumme verifiziert wird.

11. Verfahren nach Anspruch 10,
wobei die Prüfsumme eine Soll-Prüfsumme umfasst, die ausgebildet ist, einen Typ einer Magnetresonanzvorrichtung und/oder relevanter Komponenten einer Magnetresonanzvorrichtung eindeutig zu identifizieren,
wobei die Soll-Prüfsumme anhand einer Soll-Geräte-ID und dem Konfigurationsparameterdatensatz erzeugt wird.

12. Verfahren nach Anspruch 11,
wobei die Prüfsumme eine Ist-Prüfsumme umfasst, die ausgebildet ist, die Magnetresonanzvorrichtung und/oder relevante Komponenten der Magnetresonanzvorrichtung eindeutig zu identifizieren,
wobei die Ist-Prüfsumme anhand einer Ist-Geräte-ID und dem Konfigurationsparameterdatensatz (404) erzeugt wird,
wobei die Ist-Prüfsumme mit der Soll-Prüfsumme (406) verglichen wird.

13. Verfahren nach einem der vorangehenden Ansprüche,
wobei die Sicherheitseinheit (51) kontrolliert, ob ein Betriebsfehler vorliegt,
wobei der Betriebsfehler einen Softwarefehler und/oder einen Hardwarefehler und/oder einen Energieversorgungsfehler umfasst.

14. Verfahren nach einem der Ansprüche 4 oder 5,
wobei ein Funktionstest in einem abgesicherten Betriebsmodus durch die Sicherheitseinheit (51) durchgeführt wird,
wobei der abgesicherte Betriebsmodus niedrigere Grenzwerte aufweist als der eingeschränkte Betriebsmodus (S3,1) und/oder der uneingeschränkte Betriebsmodus (S3,0),
wobei der Betriebsmodus vom abgesicherten Betriebsmodus in den eingeschränkten Betriebsmodus (S3,1) wechselt, wenn der Funktionstest erfolgreich abgeschlossen wird.

15. Magnetresonanzvorrichtung (10) mit einem ersten Teil (SP) und einem zweiten Teil (nSP),
wobei der erste Teil (SP) eine Sicherheitseinheit (51) umfasst,
wobei der erste Teil (SP) ausgebildet ist, getrennt von dem zweiten Teil (nSP) betrieben zu werden,
wobei der zweite Teil (nSP) eine Systemsteuereinheit (22) zur Steuerung der Magnetresonanzvorrichtung (10) aufweist,
wobei die Sicherheitseinheit (51) ausgebildet ist, Kontrollsignale (SC) an die Systemsteuereinheit (22) des zweiten Teils (nSP) zu senden,
wobei die Sicherheitseinheit (51) ausgebildet ist, bei einer Untersuchung eines Implantat-Trägers die Magnetresonanzvorrichtung (10) in einem eingeschränkten Betriebsmodus auf Einhaltung Implantats-konformer Grenzwerte zu kontrollieren, **dadurch gekennzeichnet,**
**dass** der erste Teil (SP) Software umfasst, die derart programmiert ist, dass sie Sicherheitsanforderungen gemäß einer ersten Sicherheitskategorie erfüllt und der zweite Teil (nSP) Software umfasst, die derart programmiert ist, dass sie Sicherheitsanforderungen gemäß einer zweiten Sicherheitskategorie erfüllt,
wobei die erste Sicherheitskategorie höhere Sicherheitsanforderungen als die zweite Sicherheitskategorie aufweist.

16. Magnetresonanzvorrichtung (10) nach Anspruch 15, die ausgebildet ist, ein Verfahren nach einem der Ansprüche 2 bis 14 auszuführen.

**Claims**

1. Method for operating a magnetic resonance apparatus (10) by means of a safety unit (51), taking into account persons fitted with implants (15),
wherein the magnetic resonance apparatus (10) comprises a first part (SP) and a second part (nSP),
wherein the first part (SP) is operated separately from the second part (nSP) and comprises the safety unit (51),
wherein the second part (nSP) has a system control unit (22) for controlling the magnetic resonance apparatus (10),
wherein the safety unit (51) is embodied to send control signals (SC) to the system control unit (22) of the second part (nSP),
wherein, during an examination of a person fitted with an implant, the safety unit (51) controls the magnetic resonance apparatus (10) in a restricted operating mode to comply with implant-conformant limit values,
**characterised in that** the first part (SP) is operated using software that fulfils safety requirements in accordance with a first safety category,

wherein the second part (nSP) is operated using software that fulfils safety requirements in accordance with a second safety category,

wherein the first safety category has higher safety requirements than the second safety category.

2. Method according to claim 1,

wherein the first part (SP) comprises a switching unit (52), wherein the safety unit (51) is provided with switching information (S1) from a number of possible items of switching information by the switching unit (52),

wherein an operating mode (S3) is set by the safety unit (51) from a number of possible operating modes, depending on the switching information (S1) provided.

3. Method according to claim 2,

wherein the second part (nSP) comprises a patient registration unit (26),

wherein the safety unit (51) is provided with patient registration information (S2) by the patient registration unit (26),

wherein a change of operating mode (S3) is initiated at least partly by the patient registration information (S2) provided.

4. Method according to claim 3,

wherein the number of possible operating modes (S3) comprises an unrestricted operating mode (S3.0) and the restricted operating mode (S3.1),

wherein the number of possible items of switching information (S1) comprises active switching information (S1.1) and passive switching information (S1.0),

wherein the unrestricted operating mode (S3.0) is set if, at the time the patient registration information (S2) is provided, the switching information (S1) provided is the passive switching information (S1.0).

5. Method according to claim 4,

wherein the restricted operating mode (S3.1) is set if, at the time the patient registration information (S2) is provided, the switching information (S1) provided is the active switching information (S1.1) or if the switching information (S1) provided is the active switching information (S1.1).

6. Method according to one of claims 2 to 5,

wherein the first part (SP) comprises a display unit (53), which displays the operating mode (S3).

7. Method according to one of the preceding claims,

wherein the safety unit (51) is provided with safety measurement data (SM) during the examination, on the basis of which the check for compliance with the implant-conformant limit values is made,

wherein the safety measurement data (SM) provided is at least partly verified.

8. Method according to one of the preceding claims,

wherein the safety unit (51) is provided with at least one configuration parameter dataset (404), on the basis of which the check for compliance with the implant-conformant limit values is made.

9. Method according to claim 8,

wherein the at least one configuration parameter dataset (404) comprises a number of configuration parameter datasets, wherein one configuration parameter dataset (404) is selected from the number of configuration parameter datasets depending on parameters of the magnetic resonance apparatus (10).

10. Method according to one of claims 8 or 9,

wherein the at least one configuration parameter dataset (404) is transmitted at least in part from the second part (nSP) into the first part (SP),

wherein the at least one configuration parameter dataset (404) provided is verified on the basis of a checksum.

11. Method according to claim 10,

wherein the checksum comprises a required checksum, which is embodied to identify uniquely a type of magnetic resonance apparatus and/or relevant components of a magnetic resonance apparatus,

wherein the required checksum is created on the basis of a required device ID and the configuration parameter dataset.

12. Method according to claim 11,

wherein the checksum comprises an actual checksum, which is embodied to identify uniquely the magnetic resonance apparatus and/or relevant components of the magnetic resonance apparatus,
wherein the actual checksum is created on the basis of an actual device ID and the configuration parameter dataset (404),
wherein the actual checksum is compared with the required checksum (406).

**13.** Method according to one of the preceding claims,
wherein the safety unit (51) checks whether an operating error is present,
wherein the operating error comprises a software error and/or a hardware error and/or an energy supply error.

**14.** Method according to one of claims 4 or 5,
wherein a function test is carried out by the safety unit (51) in a safe operating mode,
wherein the safe operating mode has lower limit values than the restricted operating mode (S3.1) and/or the unrestricted operating mode (S3.0),
wherein the operating mode changes from the safe operating mode into the restricted operating mode (S3.1) when the function test is successfully concluded.

**15.** Magnetic resonance apparatus (10) with a first part (SP) and a second part (nSP),
wherein the first part (SP) comprises a safety unit (51), wherein the first part (SP) is embodied to be operated separately from the second part (nSP),
wherein the second part (nSP) has a system control unit (22) for controlling the magnetic resonance apparatus (10),
wherein the safety unit (51) is embodied to send control signals (SC) to the system control unit (22) of the second part (nSP),
wherein, during an examination of a person fitted with an implant, the safety unit (51) is embodied to control the magnetic resonance apparatus (10) in a restricted operating mode to comply with implant-conformant limit values,
**characterised in that**
the first part (SP) comprises software that is programmed such that it fulfils safety requirements in accordance with a first safety category,
and the second part (nSP) comprises software that is programmed such that it fulfils safety requirements in accordance with a second safety category,
wherein the first safety category has higher safety requirements than the second safety category.

**16.** Magnetic resonance apparatus (10) according to claim 15, which is embodied to carry out a method according to one of claims 2 to 14.

## Revendications

**1.** Procédé pour faire fonctionner une installation (10) à résonance magnétique, en tenant compte de personnes (15) portant des implants au moyen d'une unité (51) de sécurité,
dans lequel l'installation (10) à résonance magnétique comprend une première partie (SP) et une deuxième partie (nSP),
dans lequel on fait fonctionner la première partie (SP) séparément de la deuxième partie (nSP) et la première partie (SP) comprend l'unité (51) de sécurité,
dans lequel la deuxième partie (nSP) a une unité (22) de commande de système pour la commande de l'installation (10) à résonance magnétique,
dans lequel l'unité (51) de sécurité est constituée pour envoyer des signaux (SC) de contrôle à l'unité (22) de commande de système de la deuxième partie (nSP),
dans lequel, lors de l'examen d'une personne portant des implants, l'unité (51) de sécurité contrôle l'installation (10) à résonance magnétique dans un mode de fonctionnement limité de maintien de valeurs limites conformes aux implants,
**caractérisé en ce que** l'on fait fonctionner la première partie (SP) par un logiciel, qui satisfait des exigences de sécurité suivant une première catégorie de sécurité,
dans lequel on fait fonctionner la deuxième partie (nSP) par un logiciel, qui satisfait des exigences de sécurité suivant une deuxième catégorie de sécurité,
dans lequel la première catégorie de sécurité a des exigences de sécurité plus grandes que la deuxième catégorie de sécurité.

**2.** Procédé suivant la revendication 1,

dans lequel la première partie (SP) comprend une unité (52) de commutation,

dans lequel on met à disposition de l'unité (51) de sécurité par l'unité (52) de commutation une information (S1) de commutation composée de plusieurs informations de commutation possibles,

dans lequel on règle par l'unité (51) de sécurité un mode (S3) de fonctionnement parmi plusieurs modes de fonctionnement possibles en fonction de l'information (S1) de commutation mise à disposition.

3. Procédé suivant la revendication 2,

dans lequel la deuxième partie (nSP) comprend une unité (26) d'enregistrement de patient,

dans lequel on met à disposition de l'unité (51) de sécurité, par l'unité (26) d'enregistrement de patient, une information (S2) d'enregistrement de patient,

dans lequel on déclenche un changement du mode (S3) de fonctionnement au moins en partie par l'information (S2) d'enregistrement de patient mise à disposition.

4. Procédé suivant la revendication 3,

dans lequel les plusieurs modes (S3) de fonctionnement possibles comprennent un mode (S3,0) de fonctionnement illimité et le mode (S3,1) de fonctionnement limité,

dans lequel les informations (S1) de commutation possibles comprennent une information (S1,1) de commutation active et une information (S1,0) de commutation passive,

dans lequel on établit le mode (S3,0) de fonctionnement illimité si, à l'instant de la mise à disposition de l'information (S2) d'enregistrement de patient, l'information (S1) de commutation mise à disposition est l'information (S1,0) de commutation passive.

5. Procédé suivant la revendication 4,

dans lequel on établit le mode (S3,1) de fonctionnement limité si, à l'instant de la mise à disposition de l'information (S2) d'enregistrement de patient, l'information (S1) de commutation mise à disposition est l'information (S1,1) de commutation active ou si l'information (S1) de commutation mise à disposition est l'information (S1,1) de commutation active.

6. Procédé suivant l'une des revendications 2 à 5,

dans lequel la première partie (SP) comprend une unité (53) d'affichage, qui indique le mode (S3) de fonctionnement.

7. Procédé suivant l'une des revendications précédentes,

dans lequel on met à disposition de l'unité (51) de sécurité pendant l'examen des données (SM) de mesure de sécurité, au moyen desquels s'effectue la commande du maintien des valeurs limites conformes aux implants,

dans lequel on vérifie au moins en partie les données (SM) de mesure de sécurité mises à disposition.

8. Procédé suivant l'une des revendications précédentes,

dans lequel on met à disposition de l'unité (51) de sécurité au moins un ensemble (404) de données de paramètre de configuration, au moyen desquels s'effectue la commande du maintien des valeurs limites conformes aux implants.

9. Procédé suivant la revendication 8,

dans lequel le au moins un ensemble (404) de données de paramètre de configuration comprend plusieurs ensembles de données de paramètre de configuration,

dans lequel on choisit parmi les plusieurs ensembles de données de paramètre de configuration un ensemble (404) de données de paramètre de configuration en fonction de paramètres de l'installation (10) à résonnance magnétique.

10. Procédé suivant l'une des revendications 8 ou 9,

dans lequel on transmet le au moins un ensemble (404) de données de paramètre de configuration au moins en partie de la deuxième partie (nSP) à la première partie (SP),

dans lequel on vérifie le au moins un ensemble (404) de données de paramètre de configuration mises à disposition à l'aide d'une somme de contrôle.

11. Procédé suivant la revendication 10,

dans lequel la somme de contrôle comprend une somme de contrôle de consigne, qui est constituée pour identifier de manière univoque un type d'une installation à résonnance magnétique et/ou des éléments pertinents d'une installation à résonnance magnétique, dans lequel on produit la somme de contrôle de consigne à l'aide d'une ID d'appareils de consigne et de l'ensemble de données de paramètre de configuration.

**12.** Procédé suivant la revendication 11,
dans lequel la somme de contrôle est une somme de contrôle réelle, qui est constituée pour identifier de manière univoque l'installation à résonnance magnétique et/ou des éléments pertinents de l'installation à résonnance magnétique,
dans lequel on produit la somme de contrôle réelle à l'aide d'une ID d'appareils réelle et de l'ensemble (404) de données de paramètre de configuration,
dans lequel on compare la somme de contrôle réelle à la somme (406) de contrôle de consigne.

**13.** Procédé suivant l'une des revendications précédentes,
dans lequel l'unité (51) de sécurité contrôle s'il y a un défaut de fonctionnement,
dans lequel le défaut de fonctionnement comprend un défaut de logiciel et/ou un défaut de matériel et/ou un défaut d'alimentation en énergie.

**14.** Procédé suivant l'une des revendications 4 ou 5,
dans lequel on effectue par l'unité (51) de sécurité un test de fonctionnement dans un mode de fonctionnement sécurisé,
dans lequel le mode de fonctionnement sécurisé a des valeurs limite plus petites que le mode (S3,1) de fonctionnement limité et/ou que le mode (S3,0) de fonctionnement illimité,
dans lequel on fait passer le mode de fonctionnement du mode de fonctionnement sécurisé dans le mode (S3,1) de fonctionnement limité, si l'on décide que le test de fonctionnement est couronné de succès.

**15.** Installation (10) à résonnance magnétique comprenant une première partie (SP) et une deuxième (nSP),
dans laquelle la première partie (SP) comprend une unité (51) de sécurité,
dans laquelle la première partie (SP) est constituée pour fonctionner séparément de la deuxième partie (nSP),
dans laquelle la deuxième partie (nSP) a une unité (22) de commande de système pour la commande de l'installation (10) à résonnance magnétique,
dans laquelle l'unité (51) de sécurité est constituée pour envoyer des signaux (SC) de contrôle à l'unité (22) de commande de système de la deuxième partie (nSP),
dans laquelle l'unité (51) de sécurité est constituée pour, lors d'un examen d'une personne portant des implants, contrôler le maintien de valeurs limites conformes aux implants, alors que l'installation (10) à résonnance magnétique est dans un mode de fonctionnement limité,
**caractérisée**
**en ce que** la première partie (SP) comprend un logiciel, qui est programmé de manière à satisfaire des exigences de sécurité suivant une première catégorie de sécurité,
et la deuxième partie (nSP) comprend un logiciel, qui est programmé de manière à satisfaire des exigences de sécurité d'une deuxième catégories de sécurité,
dans laquelle la première catégorie de sécurité a des exigences de sécurité plus grande que la deuxième catégorie de sécurité.

**16.** Installation (10) à résonnance magnétique suivant la revendication 15 qui est constituée pour effectuer un procédé suivant l'une des revendications 2 à 14.

FIG 1

# FIG 2

# FIG 3

FIG 4

FIG 5

FIG 6

FIG 7

SP

300

226

FIG 8

SP                                    nSP

315

320                              310

226

# FIG 9

# FIG 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2007265685 A1
- DE 102006016043 A1
- DE 202008018452 U1
- US 2012086449 A1